(19) Europäisches Patentamt
European Patent Office
Office européen des brevets

(11) EP 4 389 128 A1

(12) **EUROPEAN PATENT APPLICATION**
published in accordance with Art. 153(4) EPC

(43) Date of publication:
**26.06.2024 Bulletin 2024/26**

(21) Application number: **22858550.1**

(22) Date of filing: **19.08.2022**

(51) International Patent Classification (IPC):
***A61K 31/5377*** *(2006.01)* ***A61K 35/17*** *(2015.01)*
***A61P 35/00*** *(2006.01)* ***A61P 43/00*** *(2006.01)*

(52) Cooperative Patent Classification (CPC):
**A61K 39/4632; A61K 31/5377; A61K 39/4611; A61K 39/4613; A61K 39/4631; A61K 39/464412; A61K 39/464488; A61P 35/00; A61P 43/00;**
A61K 2239/31

(86) International application number:
**PCT/JP2022/031444**

(87) International publication number:
**WO 2023/022235 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.08.2021 JP 2021135138**

(71) Applicant: **Otsuka Pharmaceutical Co., Ltd.**
**Tokyo 101-8535 (JP)**

(72) Inventors:
- **SONE, Masayuki**
  **Osaka-shi, Osaka 540-0021 (JP)**
- **MATSUYAMA, Hironori**
  **Osaka-shi, Osaka 540-0021 (JP)**
- **TAZURU, Keisuke**
  **Osaka-shi, Osaka 540-0021 (JP)**
- **KOGUE, Yosuke**
  **Osaka-shi, Osaka 540-0021 (JP)**
- **AKAMINE, Hiroki**
  **Osaka-shi, Osaka 540-0021 (JP)**
- **SUDO, Toshiki**
  **Osaka-shi, Osaka 540-0021 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **COMBINATION DRUG**

(57) The present invention discloses administering pharmaceutical compositions in combination, one of which is a pharmaceutical composition containing a tolinapant drug, and the other of which is a pharmaceutical composition containing an immune cell modified to express a chimeric antigen receptor or a recombinant T-cell receptor.

EP 4 389 128 A1

## Description

### Technical Field

Disclosed are a compound represented by formula (1):

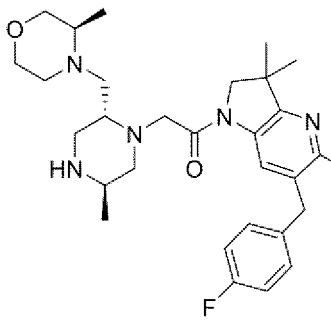

(1)

(tolinapant, IUPAC name: 1-[6-[(4-fluorophenyl)methyl]-5-(hydroxymethyl)-3,3-dimethyl-2H-pyrrolo[3,2-b]pyridin-1-yl]-2-[(2R,5R)-5-methyl-2-[[(3R)-3-methylmorpholin-4-yl]methyl]piperazin-1-yl]ethanone), a pharmaceutically acceptable salt thereof, or a pharmaceutically acceptable solvate thereof (referred to below as "tolinapant drug"); or a pharmaceutical composition comprising a modified immune cell; and associated subjects.

### Background Art

Apoptosis-inhibiting proteins (IAP) are overexpressed in many malignant tumors and involved in, for example, tumor growth. The IAP family includes eight members, which are XIAP, cIAP1, cIAP2, NAIP, ILP2, ML-IAP, survivin, and BRUCE. The IAP family members have in common 1 to 3 copies of a zinc-coordinated domain composed of about 70 amino acids, which is called "baculoviral IAP repeat (BIR) domain." The BIR domain binds to a caspase, such as caspase-3, caspase-7, or caspase-9, and functions to inhibit apoptosis.

IAP antagonists inhibit binding of caspases to the BIR domain and induce apoptosis to thereby treat tumors. Known as IAP antagonists are non-peptide mimetic small molecule antagonists, such as tolinapant (ASTX660) (see, for example, Patent Literature (PTL) 1) and second mitochondria-derived caspase activator (SMAC) mimics, such as birinapant, LCL161, and AT406.

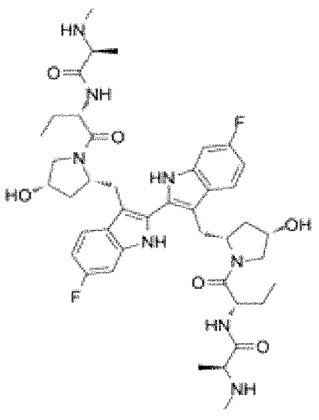

Villinapant

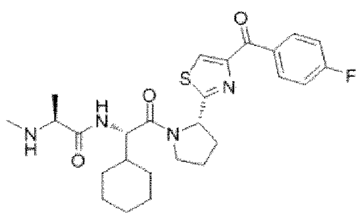

LCL161

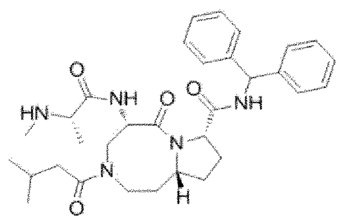

Zebinapant (AT406)

Another known approach for treating a tumor is adoptive immunotherapy using genetically modified immune cells.

For example, a method comprising introducing a nucleic acid encoding a chimeric antigen receptor into T cells collected from the living body, growing the obtained T cells in vitro, and infusing the resulting T cells is known (see, for example, Patent Literature (PTL) 2). The chimeric antigen receptor of the first generation is composed of a single-chain antibody capable of recognizing the surface antigen of tumor cells, a transmembrane domain, and an intracellular signaling domain of TCR complex CD3ζ, which activates T cells. In order to augment T-cell activation of a chimeric

antigen receptor of the first generation, there has been developed a chimeric antigen receptor of the second generation that has linked thereto an intracellular signaling domain of CD28, which is a costimulatory molecule of T cells. Further, a chimeric antigen receptor of the third generation that has linked in tandem thereto an intracellular signaling domain derived from CD137 (4-1BB) or CD134 (OX40), which are a tumor necrosis factor (TNF) receptor superfamily, has also been developed.

Further, a method comprising introducing a nucleic acid encoding a T-cell receptor capable of recognizing a specific antigen into T cells collected from the living body and growing the obtained T cells in vitro, and infusing the resulting T cells is also known. As nucleic acids that encode T-cell receptors, for example, TCR genes that target tumor antigens, such as gp100, MART-1, CEA, MAGE-A4, WT1, and NY-ESO-1, are known.

Citation List

Patent Literature

PTL 1: WO2015/092420
PTL 2: WO1993/019163

Summary of Invention

Technical Problem

An object of the present disclosure is to improve therapeutic effects of gene-modified immunotherapy.

Solution to Problem

The present inventors conducted extensive research to achieve the above object. As a result, the inventors found that when a pharmaceutical composition containing a tolinapant drug is administered in combination with a modified immune cell or when a pharmaceutical composition containing a modified immune cell is administered in combination with a tolinapant drug, the efficacy is significantly increased and a synergistic effect is provided.

The present disclosure typically includes the following.

Item 1.

A pharmaceutical composition (or drug, medicament, or IAP inhibitor) comprising

a compound represented by formula (1):

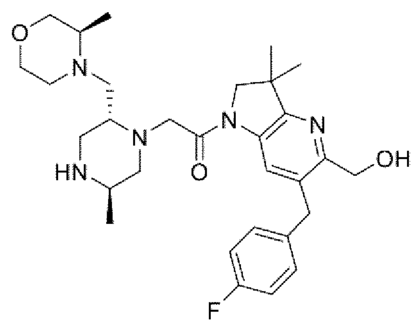

(1)

a pharmaceutically acceptable salt thereof, or
a pharmaceutically acceptable solvate thereof,

the pharmaceutical composition being administered in combination with an immune cell modified to express a chimeric antigen receptor or a recombinant T-cell receptor.

Item 1a.

The pharmaceutical composition (or drug, medicament, or IAP inhibitor) according to Item 1, which is administered before, simultaneously with, or after administration of the immune cell.

Item 2.

A pharmaceutical composition comprising an immune cell modified to express a chimeric antigen receptor or a recombinant T-cell receptor, the pharmaceutical composition being administered in combination with a compound represented by the following formula (1):

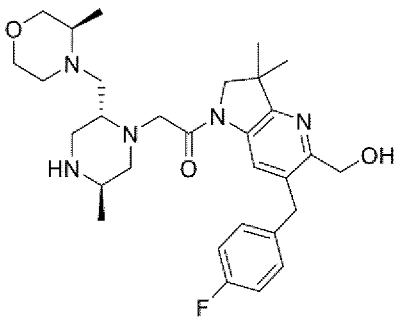

(1)

,

a pharmacologically acceptable salt thereof, or
a pharmacologically acceptable solvate thereof.

Item 2a.

The pharmaceutical composition according to Item 2, which is administered before, simultaneously with, or after administration of the compound represented by formula (1), a pharmaceutically acceptable salt thereof, or a pharmaceutically acceptable solvate thereof.

Item 3.

The pharmaceutical composition according to Item 1 or 2 or Item 1a or 2a, wherein the chimeric antigen receptor or the recombinant T-cell receptor specifically recognizes an antigen selected from the group consisting of CD19, integrin β7, and NY-ESO-1.

Item 4.

The pharmaceutical composition according to Item 1 or 2 or Item 1a or 2a, wherein the chimeric antigen receptor or recombinant T-cell receptor specifically recognizes activated integrin β7.

Item 5.

The pharmaceutical composition according to any one of Items 1 to 4, 1a, and 2a, wherein

the immune cell is a T cell or NK cell modified to express a chimeric antigen receptor, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 6.

The pharmaceutical composition according to Item 5, wherein the extracellular domain is capable of recognizing

CD19 or integrin β7.

Item 7.

The pharmaceutical composition according to Item 5 or 6, wherein the transmembrane domain is a transmembrane domain of CD28 or CD8.

Item 8.

The pharmaceutical medicinal composition according to any one of Items 5 to 7, wherein the costimulatory molecule is CD2, CD4, CD5, CD8α, CD8β, CD28, CD134 (OX40), CD137 (4-1BB), or CD278 (ICOS).

Item 9.

The pharmaceutical composition according to Item 1 or 2 or Item 1a or 2a, wherein

the immune cell is a T cell or NK cell modified to express a recombinant T-cell receptor and
the recombinant T-cell receptor has a variable domain capable of recognizing NY-ESO-1.

Item 10.

The pharmaceutical composition according to Item 1 or 2 or Item 1a or 2a, wherein the immune cell is

a T cell modified to express a chimeric antigen receptor that specifically recognizes CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

Item 11.

The pharmaceutical composition according to any one of Items 1 to 10, 1a, and 2a for use in the prevention and/or treatment of cancer.

Item 12.

The pharmaceutical composition according to any one of Items 1 to 11, 1a, and 2a, wherein the compound represented by formula (1), a pharmaceutically acceptable salt thereof, or a pharmaceutically acceptable solvate thereof is administered at a dose of 10 to 180 mg/day.

Item 12a.

The pharmaceutical composition according to any one of Items 1 to 11, 1a, and 2a, wherein the compound represented by formula (1), a pharmaceutically acceptable salt thereof, or a pharmaceutically acceptable solvate thereof is administered at a dose lower than the dose administered if administered alone or in an amount ineffective if administered alone.

Item 13.

The pharmaceutical composition according to any one of Items 1 to 12, 1a, 2a, and 12a, wherein the immune cell is administered at a dose of $1 \times 10^3$ to $1 \times 10^8$ cells (viable cell count)/kg body weight/day.

Item 13a.

The pharmaceutical composition according to any one of Items 1 to 12, 1a, 2a, and 12a, wherein the immune cell is administered at a dose lower than the dose if administered alone or in an amount ineffective if administered alone.

Item 14.

A kit comprising a first agent and a second agent,
the first agent comprising

a compound represented by formula (1):

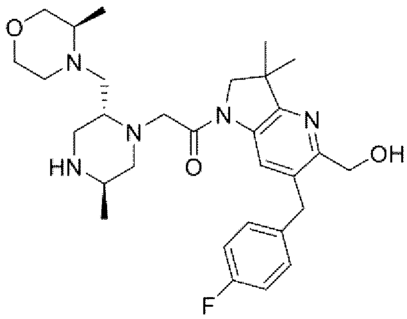

(1)

a pharmacologically acceptable salt thereof, or
a pharmaceutically acceptable solvate thereof, and

the second agent comprising an immune cell modified to express a chimeric antigen receptor or a recombinant T-cell receptor.

Item 14a.

The kit according to Item 14, wherein the first agent and the second agent are in the same formulation and administered simultaneously, or the first agent and the second agent are in different formulations and are administered simultaneously, separately, or sequentially.

Item 14b.

The kit according to Item 14 or 14a, wherein the chimeric antigen receptor or the recombinant T-cell receptor specifically recognizes an antigen selected from the group consisting of CD19, integrin β7, and NY-ESO-1.

Item 14c.

The kit according to Item 14 or 14a, wherein the chimeric antigen receptor or the recombinant T-cell receptor specifically recognizes activated integrin β7.

Item 14d.

The kit according to any one of Items 14 and 14a to 14c, wherein the immune cell is a T cell or NK cell modified to express a chimeric antigen receptor, and the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 14e.

The kit according to Item 14d, wherein the extracellular domain is capable of recognizing CD19 or integrin β7.

Item 14f.

The kit according to Item 14d or 14e, wherein the transmembrane domain is a transmembrane domain of CD28

or CD8.

Item 14g.

The kit according to any one of Items 14d to 14f, wherein the costimulatory molecule is CD2, CD4, CD5, CD8α, CD8β, CD28, CD134 (OX40), CD137 (4-1BB), or CD278 (ICOS).

Item 14h.

The kit according to claim 14 or 14a, wherein

the immune cell is a T cell or NK cell modified to express a recombinant T-cell receptor; and
the recombinant T-cell receptor has a variable domain capable of recognizing NY-ESO-1.

Item 14i.

The kit according to claim 14 or 14a, wherein the immune cell is

a T cell modified to express a chimeric antigen receptor that specifically recognizes CD19,
a T cell modified to express a chimeric antigen receptor that specifically recognizes an integrin β7,
an NK cell modified to express a chimeric antigen receptor that specifically recognizes CD19, or
a T cell modified to express a recombinant T cell receptor that specifically recognizes NY-ESO-1.

Item 14j.

The kit according to any one of Items 14 and 14a to 14i, which is for use in the prevention and/or treatment of cancer.

Item 14k.

The kit according to any one of Items 14 and 14a to 14j, wherein the compound represented by formula (1), a pharmaceutically acceptable salt thereof, or a pharmaceutically acceptable solvate thereof is administered at a dose of 10 to 180 mg/day, at a dose lower than the dose if administered alone, or in an amount ineffective if administered alone.

Item 14L.

The kit according to any one of Items 14 and 14a to 14k, wherein the immune cell is administered at a dose of $1 \times 10^3$ to $1 \times 10^8$ cells (viable cell count)/kg body weight/day, at a dose lower than the dose if administered alone, or in an amount ineffective if administered alone.

Item 15.

A method for preventing and/or treating a cancer, comprising administering to a subject in need of prevention and/or treatment of the cancer

a compound represented by formula (1):

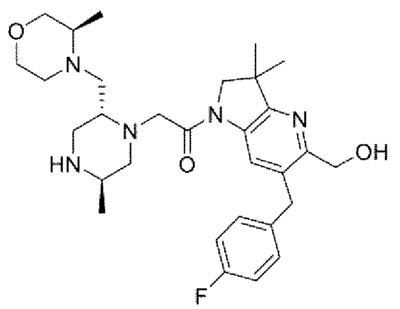

(1)

,

a pharmacologically acceptable salt thereof, or
a pharmaceutically acceptable solvate thereof,
in combination with
an immune cell modified to express a chimeric antigen receptor or a recombinant T-cell receptor.

Item 15a.

The method according to Item 15, wherein the administration is performed by administering the compound represented by formula (1), a pharmaceutically acceptable salt thereof, or a pharmaceutically acceptable solvate thereof before, simultaneously with, or after administration of the immune cells.

Item 15b.

The method according to Item 15 or 15a, wherein the chimeric antigen receptor or the recombinant T-cell receptor specifically recognizes an antigen selected from the group consisting of CD19, integrin β7, and NY-ESO-1.

Item 15c.

The method according to Item 15 or 15a, wherein the chimeric antigen receptor or the recombinant T-cell receptor specifically recognizes activated integrin β7.

Item 15d.

The method according to any one of Items 15 and 15a to 15c, wherein

the immune cell is a T cell or NK cell modified to express a chimeric antigen receptor, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 15e.

The method according to Item 15d, wherein the extracellular domain is capable of recognizing CD19 or integrin β7.

Item 15f.

The method according to Item 15d or 15e, wherein the transmembrane domain is CD28 or CD8.

Item 15g.

The method according to any one of Items 15d to 15f, wherein the costimulatory molecule is CD2, CD4, CD5, CD8α, CD8β, CD28, CD134 (OX40), CD137 (4-1BB), or CD278 (ICOS).

Item 15h.

The method according to Item 15 or 15a, wherein

the immune cell is a T cell or NK cell modified to express a recombinant T-cell receptor, and
the recombinant T-cell receptor has a variable domain capable of recognizing NY-ESO-1.

Item 15i.

The method according to Item 15 or 15a, wherein the immune cell is

a T cell modified to express a chimeric antigen receptor capable of specifically recognizing CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

Item 15j.

The method according to any one of Items 15 and 15a to 15i, wherein

the compound represented by formula (1),
a pharmaceutically acceptable salt thereof, or
a pharmaceutically acceptable solvate thereof is administered
at a dose of 10 to 180 mg/day,
at a dose lower than the dose if administered alone, or in an amount ineffective if administered alone.

Item 15k.

The method according to any one of Items 15 and 15a to 15j, wherein the immune cell is administered

at a dose of $1 \times 10^3$ to $1 \times 10^8$ cells (viable cell count)/kg body weight/day,
at a dose lower than the dose if administered alone, or in an amount ineffective if administered alone.

Item 16.

Use of a compound represented by formula (1)

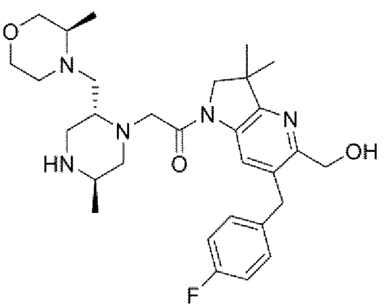

(1)

,

a pharmacologically acceptable salt thereof, or
a pharmaceutically acceptable solvate thereof
in preparing a medicament for administration in combination with an immune cell modified to express a chimeric antigen receptor or a recombinant T-cell receptor.

Item 16a.

The use according to Item 16, wherein the medicament is administered before, simultaneously with, or after administering the immune cells.

Item 16b.

The use according to Item 16 or 16a, wherein the chimeric antigen receptor or the recombinant T-cell receptor recognizes an antigen selected from the group consisting of CD19, integrin β7, and NY-ESO-1.

Item 16c.

The use according to Item 16 or 16a, wherein the chimeric antigen receptor or the recombinant T-cell receptor specifically recognizes activated integrin β7.

Item 16d.

The use according to any one of Items 16 and 16a to 16c, wherein

the immune cell is a T cell or NK cell modified to express a chimeric antigen receptor, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 16e.

The use according to Item 16d, wherein the extracellular domain is capable of recognizing CD19 or integrin β7.

Item 16f.

The use according to Item 16d or 16e, wherein the transmembrane domain is a transmembrane domain of CD28 or CD8.

Item 16g.

The use according to any one of Items 16d to 16f, wherein the costimulatory molecule is CD2, CD4, CD5, CD8α, CD8β, CD28, CD134 (OX40), CD137 (4-1BB), or CD278 (ICOS).

Item 16h.

The use according to Item 16 or 16a, wherein

the immune cell is a T cell or NK cell modified to express a recombinant T-cell receptor, and
the recombinant T-cell receptor has a variable domain capable of recognizing NY-ESO-1.

Item 16i.

The use according to Item 16 or 16a, wherein the immune cell is

a T cell modified to express a chimeric antigen receptor that specifically recognizes CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

Item 16j.

The use according to any one of Items 16 and 16a to 16i, wherein the medicament is for use in the prevention and/or treatment of cancer.

Item 16k.

The use according to any one of Items 16 and 16a to 16j, wherein the medicament is for use in administering

a compound represented by formula (1),
a pharmaceutically acceptable salt thereof, or
a pharmaceutically acceptable solvate thereof,

at a dose of 10 to 180 mg/day,
at a dose lower than the dose if administered alone, or in an amount ineffective if administered alone.

Item 16L.

The use according to any one of Items 16 and 16a to 16k, wherein the medicament is for use in administration in combination with the immune cell

at a dose of 1 × $10^3$ to 1 × $10^8$ cells (viable cell count)/kg body weight/day,
at a dose lower than the dose if administered alone, or in an amount ineffective if administered alone.

Item 17.

Use of an immune cell modified to express a chimeric antigen receptor or a recombinant T-cell receptor in preparing a medicament for administration in combination with

a compound represented by formula (1):

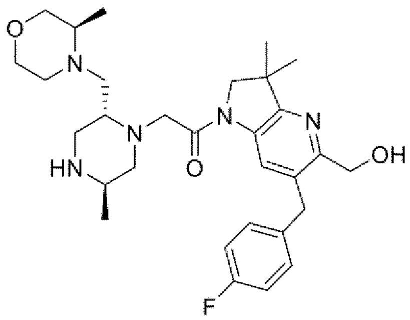

(1)

a pharmacologically acceptable salt thereof, or
a pharmaceutically acceptable solvate thereof.

Item 17a.

The use according to Item 17, wherein the medicament is for use in administration before, simultaneously with, or after administering

a compound represented by formula (1),
a pharmacologically acceptable salt thereof, or
a pharmaceutically acceptable solvate thereof.

Item 17b.

The use according to Item 17 or 17a, wherein the chimeric antigen receptor or the recombinant T-cell receptor specifically recognizes an antigen selected from the group consisting of CD19, integrin beta7, and NY-ESO-1.

Item 17c.

The use according to Item 17 or 17a, wherein the chimeric antigen receptor or the recombinant T-cell receptor specifically recognizes activated integrin β7.

Item 17d.

The use according to Item 17 and 17a to 17c, wherein

the immune cell is a T cell or NK cell modified to express a chimeric antigen receptor, and
the chimeric antigen receptor comprises

(1) an extracellular domain capable of recognizing an antigen,
(2) a transmembrane domain,

(3) an intracellular signaling domain of a costimulatory molecule, and
(4) an intracellular signaling domain of CD3ζ.

Item 17e.

The use according to Item 17d, wherein the extracellular domain is capable of recognizing CD19 or integrin β7.

Item 17f.

The use according to Item 17d or 17e, wherein the transmembrane domain is a transmembrane domain of CD28 or CD8.

Item 17g.

The use according to any one of Items 17d to 17f, wherein the costimulatory molecule is CD2, CD4, CD5, CD8α, CD8β, CD28, CD134 (OX40), CD137 (4-1BB), or CD278 (ICOS).

Item 17h.

The use according to Item 17 or 17a, wherein

the immune cell is a T cell or NK cell modified to express a recombinant T-cell receptor, and
the recombinant T-cell receptor has a variable domain capable of recognizing NY-ESO-1.

Item 17i.

The use according to Item 17 or 17a, wherein the immune cell is

a T cell modified to express a chimeric antigen receptor that specifically recognizes CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

Item 17j.

The use according to any one of Items 17 and 17a to 17i, wherein the medicament is for use in the prevention and/or treatment of cancer.

Item 17k.

The use according to any one of Items 17 and 17a to 17j, wherein the medicament is for use in administering the immune cell

at a dose of $1 \times 10^3$ to $1 \times 10^8$ cells (viable cell count)/kg body weight/day,
at a dose lower than the dose if administered alone, or in an amount ineffective if administered alone.

Item 17L.

The use according to any one of Items 17 and 17a to 17k, wherein the medicament is for use in administration in combination with

the compound represented by formula (1),
a pharmacologically acceptable salt thereof, or
a pharmaceutically acceptable solvate thereof

at a dose of 10 to 180 mg/day,
at a dose lower than the dose if administered alone, or
in an amount ineffective if administered alone.

Item 18.

A compound represented by formula (1):

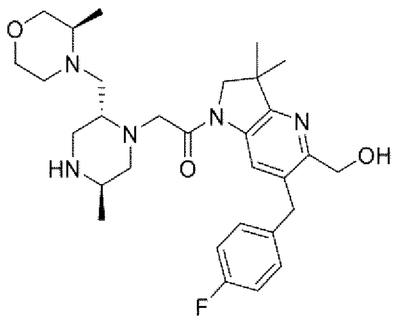

(1)

,

a pharmacologically acceptable salt thereof, or
a pharmaceutically acceptable solvate thereof,
the compound, pharmacologically acceptable salt thereof, or pharmaceutically acceptable solvate thereof being for use in administration in combination with an immune cell modified to express a chimeric antigen receptor or a recombinant T-cell receptor.

Item 18a.

The compound, pharmacologically acceptable salt thereof, or pharmaceutically acceptable solvate thereof according to Item 18,
the compound, pharmacologically acceptable salt thereof, or pharmaceutically acceptable solvate thereof being for use in administration before, simultaneously with, or after administering the immune cell.

Item 18b.

The use according to Item 18 or 18a, wherein the chimeric antigen receptor or recombinant T-cell receptor specifically recognizes an antigen selected from the group consisting of CD19, integrin β7, and NY-ESO-1.

Item 18c.

The use according to Item 18 or 18a, wherein the chimeric antigen receptor or the recombinant T-cell receptor specifically recognizes an activated integrin β7.

Item 18d.

The compound, pharmacologically acceptable salt thereof, or pharmaceutically acceptable solvate thereof according to any one of Items 18 and 18a to 18c, wherein

the immune cell is a T cell modified to express a chimeric antigen receptor or NK cells, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 18e.

The compound, pharmacologically acceptable salt thereof, or pharmaceutically acceptable solvate thereof according to Item 18d, wherein the extracellular domain is capable of recognizing CD19 or integrin β7.

Item 18f.

The compound, pharmacologically acceptable salt thereof, or pharmaceutically acceptable solvate thereof according to Item 18d or 18e, wherein the transmembrane domain is a transmembrane domain of CD28 or CD8.

Item 18g.

The compound, pharmacologically acceptable salt thereof, or pharmaceutically acceptable solvate thereof according to any one of Items 18d to 18f, wherein the costimulatory molecule is CD2, CD4, CD5, CD8α, CD8β, CD28, CD134 (OX40), CD137 (4-1BB), or CD278 (ICOS).

Item 18h.

The compound, pharmacologically acceptable salt thereof, or pharmaceutically acceptable solvate thereof according to Item 18 or 18a, wherein

the immune cell is a T cell modified to express a recombinant T-cell receptor, or NK cells; and
the recombinant T-cell receptor has a variable domain capable of recognizing NY-ESO-1.

Item 18i.

The compound, pharmacologically acceptable salt thereof, or pharmaceutically acceptable solvate thereof according to Item 18 or 18a, wherein the immune cell is

a T cell modified to express a chimeric antigen receptor capable of specifically recognizing CD19,
a T cell modified to express a chimeric antigen receptor capable of specifically recognizing integrin β7,
an NK cell modified to express a chimeric antigen receptor capable of specifically recognizing CD19, or
a T cell modified to express a recombinant T-cell receptor capable of specifically recognizing NY-ESO-1.

Item 18j.

The compound, pharmacologically acceptable salt thereof, or pharmaceutically acceptable solvate thereof according to any one of Items 18 and 18a to 18i, wherein the compound, pharmacologically acceptable salt thereof, or pharmaceutically acceptable solvate thereof is for use in the prevention and/or treatment of cancer.

Item 18k.

The compound according to any one of Items 18 and 18a to 18j, a pharmacologically acceptable salt thereof, or a pharmaceutically acceptable solvate thereof, wherein the compound, pharmacologically acceptable salt thereof, or pharmaceutically acceptable solvate thereof is for use in administration

at a dose of 10 to 180 mg/day,
at a dose lower than the dose if administered alone, or
in an amount ineffective if administered alone.

Item 18L.

The compound, pharmacologically acceptable salt thereof, or pharmaceutically acceptable solvate thereof according to any one of Items 18 and 18a to 18k, which is for use in administration in combination with the immune cell

at a dose of $1 \times 10^3$ to $1 \times 10^8$ cells (viable cell count)/kg body weight/day,
at a dose lower than the dose if administered alone, or
in an amount ineffective if administered alone.

Item 19.

An immune cell modified to express a chimeric antigen receptor or a recombinant T-cell receptor, the immune cell being for use in administration in combination with

a compound represented by formula (1):

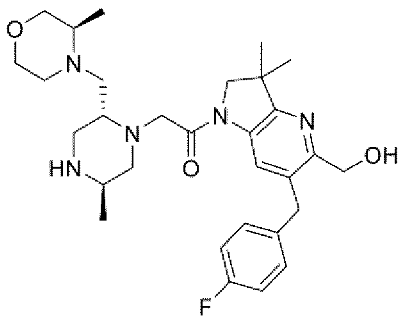

(1)

,

a pharmacologically acceptable salt thereof, or
a pharmaceutically acceptable solvate thereof.

Item 19a.

The immune cell according to Item 19, which is for use in administration before, simultaneously with, or after administration of

the compound represented by formula (1),
a pharmaceutically acceptable salt thereof, or
a pharmaceutically acceptable solvate thereof.

Item 19b.

The immune cell according to Item 19 or 19a, wherein the chimeric antigen receptor or the recombinant T-cell receptor specifically recognizes an antigen selected from the group consisting of CD19, integrin β7, and NY-ESO-1.

Item 19c.

The immune cell according to Item 19 or 19a, wherein the chimeric antigen receptor or recombinant T-cell receptor specifically recognizes an activated integrin β7.

Item 19d.

The immune cell according to any one of Items 19 and 19a to 19c, wherein

the immune cell is a T cell or NK cell modified to express a chimeric antigen receptor, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

Item 19e.

The immune cell according to Item 19d, wherein the extracellular domain is capable of recognizing CD19 or integrin β7.

Item 19f.

The immune cells according to Item 19d or 19e, wherein the transmembrane domain is a transmembrane domain of CD28 or CD8.

Item 19g.

The immune cell according to Items 19d or 19f, wherein the costimulatory molecule is CD2, CD4, CD5, CD8α, CD8β, CD28, CD134 (OX40), CD137 (4-1BB), or CD278 (ICOS).

Item 19h.

The immune cell according to Item 19 or 19a, wherein

the immune cell is a T cell or NK cell modified to express a recombinant T-cell receptor, and
the recombinant T-cell receptor has a variable domain capable of recognizing NY-ESO-1.

Item 19i.

The immune cell according to Item 19 or 19i, wherein the immune cell is

a T cell modified to express a chimeric antigen receptor that specifically recognizes CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

Item 19j.

The immune cell according to any one of Items 19 and 19a to 19i, which is for use in the prevention and/or treatment of cancer.

Item 19k.

The immune cell according to any one of Items 19 and 19a to 19j, which is for use in administration

at a dose of $1 \times 10^3$ to $1 \times 10^8$ cells (viable cell count)/kg body weight/day,
at a dose lower than the dose if administered alone, or in an amount ineffective if administered alone.

Item 19L.

The immune cell according to any one of Items 19 and 19a to 19k, which is for use in administration in combination with

a compound represented by formula (1),
a pharmacologically acceptable salt thereof, or
a pharmaceutically acceptable solvate thereof,

at a dose of 10 to 180 mg/day,
at a dose lower than the dose if administered alone, or
in an amount ineffective if administered alone.

Advantageous Effects of Invention

The present disclosure can significantly increase the efficacy of a pharmaceutical composition comprising a tolinapant drug or a modified immune cell.

Brief Description of Drawings

Fig. 1 is a graph showing a combination effect of birinapant and CD19 CAR-T.
Fig. 2 is a graph showing a combination effect of tolinapant (in the figure, tolinapant means (+)-L-lactate of a compound represented by formula (1)) and CD19 CAR-T. An asterisk (*) indicates that a combination effect in a

one-way model was tested and a combination of tolinapant and CD19 CAR-T ($1 \times 10^5$ cells/body) exhibited a significantly improved effect ($p < 0.05$) as compared to the effect achieved by controls (using only tolinapant or only CD19 CAR-T ($1 \times 10^5$ cells/body)).

Fig. 3 is a graph showing a combination effect of tolinapant (in the figure, "tolinapant" means (+)-L-lactate of a compound represented by formula (1)) and CD19 CAR-T on a low volume of tumor.

Fig. 4 is a graph showing a combination effect of LCL161 and CD19 CAR-T.

Fig. 5 is a graph showing a combination effect of AT406 and CD19 CAR-T.

Fig. 6 is a diagram showing an administration regimen.

Fig. 7 is a graph showing a combination effect of tolinapant and activated integrin β7 CAR-T.

Fig. 8 is a diagram showing an administration regimen.

Fig. 9 is a graph showing a combination effect of tolinapant and CD19 CAR-KHYG-1.

Fig. 10 is a diagram showing an administration regimen.

Description of Embodiments

A. Pharmaceutical Composition Containing a Tolinapant Drug

The pharmaceutical composition containing a tolinapant drug of the present disclosure (referred to below as "pharmaceutical composition A") is preferably a pharmaceutical composition for administration in combination with an immune cell modified to express a chimeric antigen receptor or a recombinant T-cell receptor (which may be in the form of pharmaceutical composition B described below). Specifically, the pharmaceutical composition of the present disclosure is preferably a pharmaceutical composition that is administered before, simultaneously with, or after administration of the immune cell. Pharmaceutical composition A can be typically an IAP antagonist, and in particular, can be an antagonist of XIAP, cIAP1, or cIAP2. As compared to, for example, other IAP antagonists, tolinapant drugs can more quickly exhibit efficacy when administered in combination with a modified immune cell.

A-1. Tolinapant Drug

The compound represented by formula (1) can be present as a single optical isomer in a proportion of 55% or more, 60% or more, 65% or more, 70% or more, 75% or more, 80% or more, 85% or more, 90% or more, 95% or more, 99% or more, 99.5% or more, 99.9% or more, or 100%, based on the total amount.

The pharmaceutically acceptable salt of the compound represented by formula (1) is preferably an acid addition salt. The acid addition salt may be an organic acid addition salt or an inorganic acid addition salt. Examples of acid addition salts include, but are not limited to, acetic acid, 2,2-dichloroacetic acid, adipic acid, alginic acid, ascorbic acid (e.g., L-ascorbic acid), L-asparagine acid, benzenesulfonic acid, benzoic acid, 4-acetamidobenzoic acid, butanoic acid, (+)-camphoric acid, camphor-sulfonic acid, (+)-(1S)-camphor-10-sulfonic acid, capric acid, caproic acid, caprylic acid, silicic acid, citric acid, cyclaminic acid, dodecyl sulfate, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, glucuronic acid (e.g., D-glucuronic acid), glutamic acid (e.g., L-glutamic acid), α-oxoglutaric acid, glycolic acid, hippuric acid, hydrohalogenated acids (e.g., hydrobromic acid, hydrochloric acid, and hydroiodic acid), isethionic acid, lactic acid (e.g., (+)-L-lactic acid, (-)-D-lactic acid, (±)-DL-lactic acid), lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, phosphoric acid, propionic acid, pyruvic acid, L-pyroglutamic acid, salicylic acid, 4-aminosalicylic acid, sebacic acid, stearic acid, succinic acid, sulfuric acid, tannic acid, (+)-L-tartaric acid, thiocyanic acid, p-toluenesulfonic acid, undecylenic acid, valeric acid, and acylated amino acid. In one embodiment, the pharmacologically acceptable salt of the compound represented by formula (1) is preferably a lactate (e.g., (+)-L-lactic acid), a hydrochloride (including dihydrochloride), a sulfate, or a mesylate.

Examples of the pharmacologically acceptable solvate include, but are not limited to, water, methanol, ethanol, isopropanol, acetic acid, ethyl acetate, ethanolamine, and dimethyl sulfoxide (DMSO).

A-2. Other Components

Pharmaceutical composition A preferably contains a pharmaceutically acceptable excipient as another component. Examples of pharmaceutically acceptable excipients include carriers (including solid carriers, semi-solid carriers, and liquid carriers), auxiliary agents, diluents, fillers or bulking agents, granules, coating agents, release control agents, binders, disintegrants, lubricants, preservatives, antioxidants, buffers, anti-precipitation agents, thickening agents, flavoring agents, stabilizers, surfactants, chelating agents, and other excipients commonly used in pharmaceutical compositions. Such pharmaceutically acceptable excipients can be used singly or in a combination of two or more.

A-3. Form

Pharmaceutical composition A may be a liquid formulation (e.g., injections, eye drops, nasal drops, and suspensions), a solid formulation (e.g., tablets, granules, and powders), a semi-solid formulation (e.g., ointments and suppositories), or other dosage forms known to a person skilled in the art.

A-4. Administration

Pharmaceutical composition A may be for oral administration or for parenteral administration. Pharmaceutical composition A may be for topical administration. Pharmaceutical composition A may be for intraocular, aural, intranasal, sublingual, intratracheal, intravenous, intramuscular, intraperitoneal, intrarectal, intravaginal, or transdermal administration. Pharmaceutical composition A may be for injection.

The dose of the tolinapant drug can be appropriately selected according to the purpose of administration, the subject being administered with the drug, and the subject's condition. The lower limit of the daily dose per kilogram of body weight of the subject can be, for example, 0.01 mg, 0.05 mg, 0.1 mg, 0.2 mg, 0.3 mg, 0.4 mg, 0.5 mg, 0.6 mg, 0.7 mg, 0.8 mg, 0.9 mg, or 1 mg. The upper limit of the daily dose per kilogram of body weight of the subject can also be appropriately selected as with the lower limit. The upper limit can be, for example, 10 mg, 9 mg, 8 mg, 7 mg, 6 mg, 5 mg, 4 mg, 3 mg, 2 mg, or 1 mg. The daily dose per kilogram of body weight of the subject can be, for example, in the range of 0.01 mg to 5 mg, 0.05 mg to 4 mg, or 0.1 mg to 3 mg. The daily dose can be, for example, 1 mg or more, 5 mg or more, 10 mg or more, 20 mg or more, 30 mg or more, 40 mg or more, 50 mg or more, 60 mg or more, 70 mg or more, 80 mg or more, 90 mg or more, or 100 mg or more. The daily dose can be, for example, 500 mg or less, 400 mg or less, 300 mg or less, mg or less, 200 mg or less, 190 mg or less, 180 mg or less, 170 mg or less, 160 mg or less, 150 mg or less, 140 mg or less, 130 mg or less, 120 mg or less, 110 mg or less, or 100 mg or less. The daily dose can be, for example, in the range of 1 mg to 300 mg, 5 mg to 200 mg, or 10 mg to 180 mg. When the tolinapant drug contains a salt or a solvate of tolinapant, the dose of the tolinapant drug may be an amount in terms of the weight of the free form of tolinapant (i.e., the compound represented by formula (1)) or an amount in terms of the actual weight of the salt or solvate of tolinapant. In one embodiment, when the tolinapant drug is in the form of a salt or a solvate of tolinapant, the dose of the tolinapant drug is an amount in terms of the weight of the free form of tolinapant. The daily dose may be administered in divided doses two or more times (e.g., two or three times) so that the daily dose falls within the range described above; however, the tolinapant drug is preferably administered once in a single dose. The dose of the tolinapant drug can be lower than the dose if administered alone, or can be an amount ineffective if administered alone. Even when administered in such an amount, the tolinapant drug can exhibit excellent efficacy based on its administration with a modified immune cell.

Pharmaceutical composition A can be administered at any frequency. For example, pharmaceutical composition A can be administered once, twice, or three times a day, once every two days, once every three days, once every four days, once every five days, once every six days, once a week, once every two weeks, once every three weeks, or once every four weeks in frequency. Pharmaceutical composition A may continue to be administered for a predetermined period (e.g., 1 week, 2 weeks, 3 weeks, or 4 weeks) at the frequency described above (e.g., once a day), and then the administration may be stopped for a predetermined period (e.g., 1 week, 2 weeks, 3 weeks, or 4 weeks). A cycle of the continued administration and cessation can be repeated. For example, one cycle can consist of once-a-day administration of pharmaceutical composition A for 7 days and subsequent cessation for 7 days. This cycle can be repeated once, or two or more times.

Pharmaceutical composition A may be administered before administration of the modified immune cell, such as 1 minute, 2 minutes, 3 minutes, 4 minutes, 5 minutes, 10 minutes, 20 minutes, 30 minutes, 1 hour, 2 hours, 3 hours, 4 hours, 5 hours, 6 hours, 7 hours, 8 hours, 9 hours, 10 hours, 11 hours, 12 hours, 18 hours, 24 hours, 2 days, 3 days, 4 days, 5 days, 6 days, or 1 week before the administration. Pharmaceutical composition A may be administered at the same time as the administration of the modified immune cell. Pharmaceutical composition A may be administered after administration of the modified immune cell, for example, immediately after the administration, or 1 minute, 2 minutes, 3 minutes, 4 minutes, 5 minutes, 10 minutes, 20 minutes, 30 minutes, 1 hour, 2 hours, 3 hours, 4 hours, 5 hours, 6 hours, 7 hours, 8 hours, 9 hours, 10 hours, 11 hours, 12 hours, 18 hours, 24 hours, 2 days, 3 days, 4 days, 5 days, 6 days, or 1 week after the administration. Pharmaceutical composition A may also be administered, for example, after administration of the modified immune cell and when the tumor marker level is higher than the baseline level.

For example, other chemotherapy (administration of one or more other drugs) or non-chemotherapy (e.g., radiation therapy, photodynamic therapy, surgery, and dietary restriction) may be performed between the administration of pharmaceutical composition A and the administration of a modified immune cell, or between the administration of pharmaceutical composition A and the subsequent administration of pharmaceutical composition A. Examples of such

other drugs include alkylating agents, DNA methylation inhibitors, and antimetabolites (e.g. pyrimidine antimetabolites, purine antimetabolites, and folate antimetabolite).

Examples of the subject being administered with pharmaceutical composition A include, but are not limited to, humans, dogs, cats, monkeys, sheep, horses, cattle, mice, and rats. The subject being administered with pharmaceutical composition A may also be a subject being or having been administered with a modified immune cell. The subject being administered with pharmaceutical composition A is preferably a subject in need of prevention and/or treatment of an IAP-related disease, in particular cancer (or a patient with a primary disease or a patient with a recurrent disease).

The formulation, production method, administration method, etc. for pharmaceutical composition A can be designed based on, for example, the description in WO2015/092420 (which is incorporated herein in its entirety by reference).

A-5. Pharmaceutical Application

Pharmaceutical composition A is not particularly limited in application as long as it is used for an application in which the effect (therapeutic effect in gene-modified immunotherapy) of pharmaceutical composition B can be increased. Pharmaceutical composition A can be used, for example, in prevention and/or treatment of an IAP-related disease. In the present specification, the term "prevention" is used to include the meaning of, for example, prevention of recurrence. The term "treatment" is used to include the meaning of, for example, prevention or delay of progression, amelioration, mitigation, and remission. Examples of the IAP-related disease include disorders associated with cell accumulation (e.g., cancer, autoimmune disorders, inflammation, and restenosis), disorders in which excessive apoptosis results in cell loss (e.g., neurodegeneration, such as Alzheimer's disease, Parkinson's disease, Huntington's disease, and amyotrophic lateral sclerosis, ischemia, stroke, myocardial infarction, heart failure, osteoporosis, and AIDS). In the present specification, "cancer" means tumor in a broad sense and can be used synonymously with "tumor." The IAP-related disease is preferably cancer. The cancer can be a cancer susceptible to antagonism of at least one IAP selected from the group consisting of XIAP, cIAP1, cIAP2, NAIP, ILP2, ML-IAP, survivin, and BRUCE, more specifically XIAP, cIAP1, cIAP2, and ML-IAP, even more specifically XIAP, cIAP1, and cIAP2, and most specifically XIAP. The cancer may be a relapsed or refractory cancer.

The cancer can be a solid cancer or a blood cancer. The solid cancer can be a cancer of epithelial origin, a cancer of mesenchymal origin, or other cancers.

In one embodiment, examples of solid cancers include, but are not limited to, transitional epithelial cancer; bladder and urinary tract cancers; breast cancer; cancers of the gastrointestinal tract (including the esophagus, stomach (gastric), small intestine, colon, rectum, and anus); liver cancer (e.g., hepatocellular cancer and hepatoblastoma); gallbladder and biliary cancers (e.g., bile duct cancer); pancreatic cancer (e.g., pancreatic endocrine cancers such as metastatic pancreatic cancer, and pancreatic exocrine cancers such as pancreatic ductal adenocarcinoma); kidney cancer (e.g., renal cell carcinoma and nephroblastoma); lung cancer (e.g., adenocarcinomas, small cell lung carcinomas, non-small cell lung carcinomas, bronchoalveolar epithelial carcinoma, and mesotheliomas); cancer of the head and neck (e.g., cancers of the tongue, oral cavity, larynx, pharynx, nasopharynx, tonsil, salivary glands, nasal cavity, and paranasal sinuses); ocular and adnexal cancers (e.g., retinoblastoma); cancers of the ovary, fallopian tubes, peritoneum, vagina, vulva, penis, cervix, myometrium, and endometrium; thyroid cancers (e.g., medullary thyroid cancer, papillary thyroid cancer, undifferentiated thyroid cancer, and follicular thyroid cancer); adrenal cancer (e.g., adrenocortical carcinoma); prostate cancer (e.g., hormone-refractory prostate cancer); and skin cancers (e.g., melanoma (e.g., malignant melanoma at stages 3 and 4), basal cell cancer, squamous cell carcinoma, head and neck squamous cell carcinoma (HNSCC), keratoacanthoma, dysplastic nevus, and mycosis fungoides). In one embodiment, other examples of solid cancers include, but are not limited to, Merkel cell carcinoma, metastatic non-small cell carcinoma, metastatic breast cancer, metastatic colorectal cancer, metastatic gastric cancer, metastatic non-urogenital cancer, metastatic non-small cell lung cancer, metastatic ovarian cancer, metastatic renal cell cancer, HBV infection and related cancers, and vulvar disease.

In one embodiment, other examples of solid cancers include, but are not limited to, sarcomas of soft tissue, bone, or cartilage, such as soft tissue sarcoma, osteosarcoma, fibrosarcoma, chondrosarcoma, rhabdomyosarcoma, leiomyosarcoma, liposarcoma, haemangiosarcoma, Kaposi's sarcoma, Ewing's sarcoma, synovial sarcoma, epitheliosarcoma, gastrointestinal stromal tumor, benign and malignant histiocytomas, and dermatofibrosarcoma protuberans.

In one embodiment, other examples of solid cancers include, but are not limited to, tumors of the central or peripheral nervous system (e.g., astrocytoma, glioma, glioblastoma, meningioma, brain tumor, ependymoma, pineal tumor, and schwannoma); endocrine tumors (e.g., pituitary tumor, adrenal tumor, pancreatic islet cell tumor, parathyroid tumor, carcinoid tumor, and medullary carcinoma of the thyroid); germ cell and trophoblastic tumors (e.g., teratoma, seminoma, dysgerminoma, hydatidiform mole, and choriocarcinoma); paediatric and embryonal tumors (e.g., medulloblastoma, neuroblastoma, Wilms tumor, and primitive neuroectodermal tumor); and syndromes, congenital or otherwise, that leave the patient susceptible to malignancy (e.g., xeroderma pigmentosum).

Examples of the blood cancer include, but are not limited to, lymphoid leukemia, such as acute lymphoblastic

leukemia (ALL) (e.g., B-ALL and T-ALL) and chronic lymphocytic leukemia (CLL) (e.g., hairy cell leukemia and prolymphocytic leukemia); B cell lymphomas (e.g., diffuse large B-cell lymphoma (DLBCL), primary mediastinal large B-cell lymphoma (PMBCL), follicular lymphoma, Burkitt's lymphoma, mantle cell lymphoma, and lymphoblastic leukemia); marginal zone B-cell lymphoma, high-grade B cell lymphoma, lymphoplasmacytic lymphoma, and splenic marginal zone lymphoma); T-cell lymphoma (e.g., peripheral T-cell lymphoma (PTCL), cutaneous T-cell lymphoma (CTCL), adult T-cell lymphoma, anaplastic large-cell lymphoma, angioimmunoblastic T-cell lymphoma, and Sezary syndrome); natural killer (NK) cell lymphoma; Hodgkin lymphoma (HL); non-Hodgkin lymphoma; MALT lymphoma; primary cutaneous follicle center lymphoma (PCFCL); T-cell leukemia (e.g., adult T-cell leukemia, T-cell large granular lymphocyte leukemia, and precursor T lymphoblastic leukemia); leukemia/lymphoma; adult T-cell leukemia/lymphoma (ATLL); hairy-cell leukemia; monoclonal γ-globulinemia of unknown significance; plasmacytoma; multiple myeloma (MM) and post-transplant lymphoproliferative disorder; acute myeloblastic leukemia; myeloid leukemia, such as acute myeloid leukemia (AML) and chronic myeloid leukemia (CML); chronic myelomonocytic leukemia (CMML); myeloproliferative syndromes (e.g., primary myelofibrosis hypereosinophilic syndrome, polycythemia vera, essential thrombocythemia), myelodysplastic syndrome, and promyeloid leukemia.

In one embodiment, the cancer is a blood cancer that includes, for example, acute myeloid leukemia (AML), T-cell lymphoma (peripheral T-cell lymphoma (PTCL), cutaneous T-cell lymphoma (CTCL), adult T-cell leukemia-lymphoma (ATLL), chronic myelomonocytic leukemia (CMML), acute lymphoblastic leukemia (ALL), and diffuse large B-cell lymphoma (DLBCL). In another embodiment, the cancer is a blood cancer that includes multiple myeloma.

In one embodiment, the cancer is a solid cancer that includes, for example, head and neck squamous cell carcinoma (HNSCC), prostate cancer, pancreatic cancer, small-cell lung cancer, non-small-cell lung cancer, colon cancer, melanoma, rectal cancer, cancers of the bladder and cervix, and breast cancer.

## B. Pharmaceutical Composition Containing an Immune Cell Modified to Express a Chimeric Antigen Receptor or a Recombinant T-cell Receptor

The pharmaceutical composition containing an immune cell modified to express a chimeric antigen receptor (CAR) or a recombinant T-cell receptor (TCR) (referred to below as "pharmaceutical composition B") according to the present disclosure is preferably a pharmaceutical composition for administration in combination with a tolinapant drug (which may be in the form of pharmaceutical composition A). Specifically, the pharmaceutical composition is preferably administered before, simultaneously with, or after administration of the tolinapant drug.

The chimeric antigen receptor (CAR) or the recombinant T-cell receptor (TCR) recognizes and binds to a specific antigen. The antigen is preferably a tumor antigen. Examples of the tumor antigen include, but are not limited to, 5T4, α5β1-integrin, integrin β7 (e.g., activated integrin β7), 707-AP, AFP, ART-4, B7H4, BAGE, β-catenin/m, Bcr-abl, MN/C IX antibody, CA125, CAMEL, CAP-1, CASP-8, CD4, CD19, CD20, CD22, CD25, CDC27/m, CD30, CD33, CD52, CD56, CD80, CD98, CDK4/m, CEA, CT, Cyp-B, DAM, EGFR, ErbB3, ELF2M, EMMPRIN, EpCam, ETV6-AML1, G250, GAGE, GnT-V, Gp100, HAGE, HER-2/new, HLA-A*0201-R170I, HPV-E7, HSP70-2M, HST-2, hTERT (or hTRT), iCE, IGF-1R, IL-2R, IL-5, KIAA0205, LAGE, LDLR/FUT, MAGE, MART-1/melan-A, MART-2/Ski, MC1R, myosin/m, MUC1, MUM-1, MUM-2, MUM-3, NA88-A, PAP, proteinase-3, p190 minor bcr-abl, Pml/RARα, PRAME, PSA, PSM, PSMA, RAGE, RU1, RU2, SAGE, SART-1, SART-3, survivin, TEL/AML1, TGFβ, TPI/m, TRP-1, TRP-2, TRP-2/INT2, VEGF, WT 1, NY-ESO-1, NY-ESO-B, MAGE-A1, MAGE-A3, and MAGE-A4. In one embodiment, the tumor antigen is preferably CD19, integrin β7, or NY-ESO-1. In another embodiment, the tumor antigen is preferably CD19, activated integrin β7, or NY-ESO-1.

### B-1. CAR

The CAR preferably contains an extracellular domain and an intracellular signaling domain.

### B-1-1. Extracellular Domain

The extracellular domain is not particularly limited as long as it can recognize an antigen (or binds or specifically binds to an antigen). The extracellular domain preferably contains an antigen-binding site of an antibody or an antigen-binding (or ligand-binding) site of a receptor.

The antigen-binding site of an antibody may be the entire region of an antibody or an antigen-binding fragment of an antibody. The antibody may be a monoclonal antibody or a polyclonal antibody. The antibody can also be, for example, a mouse antibody, a chimeric antibody, a humanized antibody, or a fully humanized antibody. The antigen-binding fragment is not particularly limited as long as it comprises variable regions of H (heavy) chains and L (light) chains. Examples of the antigen-binding fragment include, but are not limited to, Fv, Fab, Fab', $(Fab')_2$, scFv, scFv-Fc, diabody, triabody, tetrabody, minibody, and nanobody.

The antigen-binding site of the receptor may be the entire region of the receptor or may be an antigen-binding domain of the receptor. Examples of the receptor include TCRs, such as TCRα, TCRβ, TCRγ, and TCR5; T-cell co-receptors, such as CD4, CD8α, CD8β, CD11A, CD11B, CD11C, CD18, CD29, CD41, CD49A, CD49B, CD49D, CD49E, CD49F, CD51, and CD61; and natural cytotoxicity receptors (NCR) of NK cells, such as NKp30, NKp44, and NKp46.

In one embodiment, the extracellular domain preferably contains an antigen-binding site of an antibody, more preferably scFv, and even more preferably scFv derived from anti-human CD19 antibody (e.g., FMC63) or anti-human activated integrin β7 antibody (e.g., MMG49). The scFv can be prepared by a commonly used method. Examples of the method include, but are not limited to, methods described in U.S. Patent No. 4694778; Science, vol. 242, pp. 423-442 (1988); Nature, vol. 334, p. 54454 (1989); Science, vol. 242, pp. 1038-1041 (1988); and Molecular Immunology, Vol. 34, No. 16-17, pp. 1157-1165 (1997).

The antigen that an extracellular domain can recognize or to which an extracellular domain can bind is preferably a tumor antigen. Examples of the tumor antigen include, but are not limited to, 5T4, α5β1-integrin, integrin β7 (e.g., activated integrin β7), 707-AP, AFP, ART-4, B7H4, BAGE, β-catenin/m, Bcr-abl, MN/C IX antibody, CA125, CAMEL, CAP-1, CASP-8, CD4, CD19, CD20, CD22, CD25, CDC27/m, CD30, CD33, CD52, CD56, CD80, CD98, CDK4/m, CEA, CT, Cyp-B, DAM, EGFR, ErbB3, ELF2M, EMMPRIN, EpCam, ETV6-AML1, G250, GAGE, GnT-V, Gp100, HAGE, HER-2/new, HLA-A*0201-R170I, HPV-E7, HSP70-2M, HST-2, hTERT (or hTRT), iCE, IGF-1R, IL-2R, IL-5, KIAA0205, LAGE, LDLR/FUT, MAGE, MART-1/melan-A, MART-2/Ski, MC1R, myosin/m, MUC1, MUM-1, MUM-2, MUM-3, NA88-A, PAP, proteinase-3, p190 minor bcr-abl, Pml/RARα, PRAME, PSA, PSM, PSMA, RAGE, RU1, RU2, SAGE, SART-1, SART-3, survivin, TEL/AML1, TGFβ, TPI/m, TRP-1, TRP-2, TRP-2/INT2, VEGF, WT1, NY-ESO-1, and NY-ESO-B. In one embodiment, the tumor antigen is preferably CD19 or integrin β7. In another embodiment, the tumor antigen is preferably CD19 or activated integrin β7. In a further different embodiment, the tumor antigen is preferably activated integrin β7. CAR capable of recognizing or binding to CD19 is referred to as "CD19 CAR." The T cell and NK cell modified to express CD19 CAR are referred to as "CD19 CAR-T" and "CD19 CAR-NK," respectively. Examples of CD19 CAR-NK include CD19 CAR-KHYG-1. Examples of CAR capable of recognizing or binding to activated integrin β7 include activated integrin β7 CAR. The T cell modified to express activated integrin β7 CAR is referred to as "activated integrin β7 CAR-T (aITGB7 CAR-T)."

In one embodiment, the extracellular domain preferably comprises a heavy-chain CDR 1 having the amino acid sequence of SEQ ID NO: 1 (GVSLPDYG), a heavy-chain CDR 2 having the amino acid sequence of SEQ ID NO: 2 (IWGSETT), a heavy-chain CDR 3 having the amino acid sequence of SEQ ID NO: 3 (AKHYYYGGSYAMDY), a light-chain CDR1 having the amino acid sequence of SEQ ID NO: 4 (QDISKY), a light-chain CDR 2 having the amino acid sequence of SEQ ID NO: 5 (HTS), and a light-chain CDR3 having the amino acid sequence of SEQ ID NO: 6 (QQGNTLPYT). In the present specification, heavy-chain CDRs 1 to 3 and light-chain CDRs 1 to 3 are identified by homology searches using IMGT/BlastSearch (http://www.imgt.org/blast/).

Further, the extracellular domain preferably contains an antigen-binding site (in particular, scFv) of an antibody that competitively inhibits binding, to CD19, of an antibody comprising a heavy-chain CDR 1 having the amino acid sequence of SEQ ID NO: 1, a heavy-chain CDR2 having the amino acid sequence of SEQ ID NO: 2, a heavy-chain CDR3 having the amino acid sequence of SEQ ID NO: 3, a light-chain CDR1 having the amino acid sequence of SEQ ID NO: 4, a light-chain CDR2 having the amino acid sequence of SEQ ID NO: 5, and a light-chain CDR3 having the amino acid sequence of SEQ ID NO: 6.

In one embodiment, the extracellular domain preferably comprises an antigen-binding site (in particular, scFv) of an antibody that competitively inhibits binding, to CD19, of an antibody comprising a heavy-chain CDR1 having the amino acid sequence of SEQ ID NO: 7 (GYTFSSYW), a heavy-chain CDR 2 having the amino acid sequence of SEQ ID NO: 8 (MLPGSGSS), a heavy-chain CDR3 having the amino acid sequence of SEQ ID NO: 9 (ARGDGNYWYFDV), a light-chain CDR1 having the amino acid sequence of SEQ ID NO: 10 (SSVGY), a light-chain CDR 2 having the amino acid sequence of SEQ ID NO: 11 (ATS), and a light-chain CDR3 having the amino acid sequence of SEQ ID NO: 12 (QQWSSDPPT).

Further, the extracellular domain preferably comprises a heavy-chain CDR 1 having the amino acid sequence of SEQ ID NO: 7, a heavy-chain CDR2 having the amino acid sequence of SEQ ID NO: 8, a heavy-chain CDR3 having the amino acid sequence of SEQ ID NO: 9, a light-chain CDR1 having the amino acid sequence of SEQ ID NO: 10, a light-chain CDR2 having the amino acid sequence of SEQ ID NO: 11, and a light-chain CDR3 having the amino acid sequence of SEQ ID NO: 12.

In one embodiment, the extracellular domain preferably comprises an antigen-binding site of an antibody having an epitope in the region consisting of the 20th to 109th amino acid residues of activated human integrin β7. Preferably, the antibody has an affinity for activated human integrin β7 and binds to myeloma cells but does not bind to normal blood cells. The activated integrin β7 CAR-T (aITGB7 CAR-T) in the present invention is described in WO2017/026331 or Nature Medicine 23 12 2007 1436, which are incorporated herein in their entireties by reference.

In the present specification, the "identity" of an amino acid sequence refers to the degree of amino acid identity when two or more amino acid sequences to be compared are optimally aligned. The amino acid sequence identity can

be calculated, for example, using default parameters in the homology algorithm BLAST (Basic Local Alignment Search Tool) (http://www.ncbi.nlm.nih.gov/BLAST/) of the National Centre for Biotechnology Information (NCBI). The "conservative substitution" means that one or more amino acids are replaced with one or more other amino acids in such a manner that the function or property of the protein is substantially not changed. Examples of the conservative substitution include, but are not limited to, substitution of a group of non-polar amino acids (hydrophobic amino acids): alanine (A), valine (V), leucine (L), isoleucine (I), proline (P), phenylalanine (F), methionine (M), and tryptophan (W); substitution of a group of polar amino acids (neutral amino acids): glycine (G), asparagine (N), glutamine (Q), serin (S), threonine (T), tyrosine (Y), and cysteine (C); substitution of a group of acidic amino acids: asparagine acid (D) and glutamic acid (E); and substitution of basic amino acids: lysine (K), arginine (R)), and histidine (H).

The equilibrium dissociation constant ($K_D$), which represents binding affinity of the extracellular domain to the antigen is, for example, 1 μM or less, 500 nM or less, or 100 nM or less, and 0.1 pM or more, 0.5 pM or more, or 1 pM or more. The equilibrium dissociation constant can be determined, for example, by SPR measurement or Biacore (trademark) analysis.

The extracellular domain may contain, in addition to an antigen-binding site of an antibody or an antigen-binding (ligand-binding) site of a receptor, extracellular domains of other proteins. Examples of such other proteins include, but are not limited to, T-cell co-receptors, such as CD3ζ, CD3ε, CD4, CD5, CD8, CD9, CD16, CD22, CD28, CD33, CD37, CD64, CD80, CD86, CD134 (OX40), CD137, CD154, and CD278 (ICOS). In one embodiment, the extracellular domain of such other proteins is preferably an extracellular domain of CD28. In another embodiment, the extracellular domain of another protein is the amino acid sequence of SEQ ID NO: 13: IEVMYPPPYLDNEKSNGTIIHVKGKHLCPSPLFPGPSKP, an amino acid sequence having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 13, or an amino acid sequence having deletion, substitution (e.g., conservative substitution), insertion and/or addition of 1 to 3 amino acids in the amino acid sequence of SEQ ID NO: 13.

B-1-2. Intercellular Signaling Domain

The intracellular signaling domain is not particularly limited as long as when the extracellular domain recognizes an antigen, signals can be transmitted into the cells. In one embodiment, the intracellular signaling domain preferably has an antigen-dependent primary activation sequence (a primary cytoplasmic signaling sequence) and an antigen-independent secondary activation or co-stimulatory sequence (secondary cytoplasmic signaling sequences).

The primary cytoplasmic signaling sequence may have a signaling motif known as an immune-receptor tyrosine-based activation motif (ITAM) (Nature, vol. 338, pp. 383-384 (1989)) and/or may have a signaling motif known as an immune receptor tyrosine-based inhibitory motif (ITIM) (J Immunol., vol. 162, No. 2, pp. 897-902 (1999)).

The intracellular signaling domain having a primary cytoplasmic signaling sequence preferably has one or more ITAMs or preferably has an exogenous STAT3-related motif. Examples of the intracellular signaling domain include, but are not limited to, intracellular signaling domains of CD3ζ, FcRγ, FcRβ, CD3γ, CD3δ, CD3ε, CD5, CD22, CD79a, CD79b, and CD66d. In one embodiment, the intracellular signaling domain is preferably an intracellular signaling domain of CD3ζ. In another embodiment, the intracellular signaling domain is preferably the amino acid sequence of SEQ: 14:

RVKFSRSADAPAYQQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPR,

an amino acid sequence having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 14, or

an amino acid sequence having deletion, substitution (e.g., conservative substitution), insertion and/or addition of 1 to 3 amino acids in the amino acid sequence.

The intracellular signaling domain having a secondary cytoplasmic signaling sequence is preferably an intracellular signaling domain of one or more co-stimulatory molecules. Examples of co-stimulatory molecules include, but are not limited to, CD2, CD4, CD5, CD8α, CD8β, CD28, CD134 (OX40), CD137 (4-1BB), CD154, and CD278 (ICOS). In one embodiment, the intracellular signaling domain is preferably an intracellular signaling domain of CD28 and/or an intracellular signaling domain of CD137 (4-1BB), and more preferably an intracellular signaling domain of CD28. In another embodiment, the intracellular signaling domain is preferably the amino acid sequence of SEQ ID NO: 15:

RSKRSRLLHSDYMNMTPRRPGPTRKHYQPYAPPRDFAAYRS,
an amino acid sequence having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 15, or
an amino acid sequence having deletion, substitution (e.g., conservative substitution), insertion and/or addition of 1 to 3 amino acids in the amino acid sequence of SEQ ID NO: 15.

CAR may further have an additional intracellular signaling domain other than those mentioned above. For example, CAR may have a domain that induces the expression of a cytokine (e.g., IL-12). The CAR may also contain an intracellular domain (e.g., IL-2R β chain fragment) of the cytokine receptor. The additional intracellular signaling domain may be inserted, for example, between an intracellular signaling domain of a costimulatory molecule and a primary cytoplasmic signaling sequence.

B-1-3. Transmembrane Domain

The CAR preferably contains a transmembrane domain between the extracellular domain and the intracellular signaling domain. The transmembrane domain can be derived from any polypeptide and can have one or more transmembrane sequences. The polypeptide can be a natural polypeptide or an artificial polypeptide.
Examples of the natural polypeptide-derived transmembrane domain include TCRs, such as TCRa and TCRβ; T-cell co-receptors, such as CD3ζ, CD3ε, CD4, CD5, CD8, CD9, CD16, CD22, CD28, CD33, CD37, CD64, CD80, CD86, CD134 (OX40), CD137, CD154, and CD278 (ICOS); and transmembrane domains of TNF receptors, such as GITR.
Preferably, the transmembrane domain derived from an artificial polypeptide mainly comprises a polypeptide containing a hydrophobic residue, such as leucine and valine. The transmembrane domain preferably has a triplet of phenylalanine, tryptophan, and valine at each end.
In one embodiment, the transmembrane domain is preferably a transmembrane domain of CD28 or CD8, and more preferably a transmembrane domain of CD28. In another embodiment, the transmembrane domain is the amino acid sequence of SEQ ID NO: 16:

FWVLVVVGGVLACYSLLVTVAFIIFWV,
an amino acid sequence having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 16, or
an amino acid sequence having deletion, substitution (e.g., conservative substitution), insertion and/or addition of 1 to 3 amino acids in the amino acid sequence of SEQ ID NO: 16.

B-1-4. Linker Sequence or Spacer Domain

The CAR may have a linker sequence or a spacer domain between an extracellular domain and a transmembrane domain or between a transmembrane domain and an intracellular signaling domain.
As long as the linker sequence or the spacer domain has the function of connecting two domains, there is no particular limitation. The linker sequence preferably comprises 10 or less amino acids, and preferably comprises 2 and more amino acids. The spacer domain preferably comprises 300 amino acids or less, 200 amino acids or less, 100 amino acids or less, or 50 amino acids or less, and preferably contains 10 amino acids or more, 15 amino acids or more, 20 amino acids or more, or 25 amino acids or more.
The linker sequence is preferably selected from a glycine-serine continuous sequence, the amino acid sequence of SEQ ID NO: 17 (GSTSGSGKPGSGEGSTKG), and the amino acid sequence of SEQ ID NO: 18 (GSTSGGGSGGGSGGGGSS).
The spacer domain preferably has a sequence that promotes binding of CAR and an antigen and that enhances signaling to cells. The sequence preferably contains, for example, cysteine, charged amino acids, or serine or threonine within a potential glycosylation site. Examples of the spacer domain include, but are not limited to, a portion of CD8α, CD8β, CD28, or IgG4 (e.g. a hinge region).
The CAR may comprise a leader sequence (a signal peptide sequence) at the N-terminus. The signal peptide sequence preferably comprises 15 or more amino acids and preferably comprises 30 or less amino acids. Examples of the signal peptide include, but are not limited to, proteins having an intracellular signaling domain. The signal peptide can be, for example, GM-CSFR signal peptide, oncostatin M signal peptide, or a signal peptide, such as CD8a, IL-2, or IL-3. Alternatively, the signal peptide can be an Ig heavy-chain signal sequence (VHCAMP). The signal peptide may be derived from a human or non-human source, such as insect cells or viruses. In one embodiment, the leader sequence (signal peptide sequence) is preferably the amino acid sequence of SEQ ID NO: 19:

MLLLVTSLLLCELPHPAFLLIP,

an amino acid sequence having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 19, or

an amino acid sequence having deletion, substitution (e.g., conservative substitution), insertion and/or addition of 1 to 3 amino acids in the amino acid sequence of SEQ ID NO: 19.

In one embodiment, CAR preferably comprises, in order from the N-terminal side, scFv derived from anti-human CD19 antibody (e.g., FMC63) or anti-human activated integrin β7 antibody (e.g., MMG49), CD28 extracellular domain, CD28 transmembrane domain, CD28 intracellular signaling domain, and CD3ζ intracellular signaling domain. CAR preferably further contains GM-CSFR signal peptide at the N-terminal side of scFv derived from anti-human CD19 antibody (e.g., FMC63) or anti-human activated integrin β7 antibody (e.g., MMG49).

In another embodiment, CAR is preferably the amino acid sequence of SEQ ID NO: 20 (GenBank Accession No. HM852952.1):

MLLLVTSLLLCELPHPAFLLIPDIQMTQTTSSLSASLGDRVTISCRASQDISKYLNWYQQKPDGT
VKLLIYHTSRLHSGVPSRFSGSGSGTDYSLTISNLEQEDIATYFCQQGNTLPYTFGGGTKLEITG
STSGSGKPGSGEGSTKGEVKLQESGPGLVAPSQSLSVTCTVSGVSLPDYGVSWIRQPPRKGLEWL
GVIWGSETTYYNSALKSRLTIIKDNSKSQVFLKMNSLQTDDTAIYYCAKHYYYGGSYAMDYWGQG
TSVTVSSAAAIEVMYPPPYLDNEKSNGTIIHVKGKHLCPSPLFPGPSKPFWVLVVVGGVLACYSL
LVTVAFIIFWVRSKRSRLLHSDYMNMTPRRPGPTRKHYQPYAPPRDFAAYRSRVKFSRSADAPAY
QQGQNQLYNELNLGRREEYDVLDKRRGRDPEMGGKPRRKNPQEGLYNELQKDKMAEAYSEIGMKG
ERRRGKGHDGLYQGLSTATKDTYDALHMQALPPR,

an amino acid sequence comprising the amino acid sequences of SEQ ID NOs: 1 to 6 and having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 20, or

an amino acid sequence comprising the amino acid sequences of SEQ ID NOs: 1 to 6 and having deletion, substitution (e.g., conservative substitution), insertion and/or addition of 1 to 3 amino acids in the amino acid sequence of SEQ ID NO: 20.

Further, in another embodiment, CAR is preferably the amino acid sequence of SEQ ID NO: 21:

MDFQVQIFSFLLISASVIMSRGQIVLSQSPAILSASPGEKVTMTCRASSSVGYMHWFQQKPGSSP
KPWIYATSNLASGVPARFSGSESGTSYSLTISRVEAEDAATYYCQQWSSDPPTFGGGTKLEIKRG
STSGSGKPGSGEGSQVQLQQSGAELMKPGASVKISCKASGYTFSSYWIEWVKQRPGHGLEWIGEM
LPGSGSSNYNEKFKGKATFTADTSSNTAYMQLSSLTSEDSAVYYCARGDGNYWYFDVWGAGTTVT
VSSAAAIEVMYPPPYLDNEKSNGTIIHVKGKHLCPSPLFPGPSKPFWVLVVVGGVLACYSLLVTV
AFIIFWVRSKRSRLLHSDYMNMTPRRPGPTRKHYQPYAPPRDFAAYRSRVKFSRSADAPAYQQGQ
NQLYNELNLGRREEYDVLDKRRGRDPEMGGKPRRKNPQEGLYNELQKDKMAEAYSEIGMKGERRR
GKGHDGLYQGLSTATKDTYDALHMQALPPR,

an amino acid comprising the amino acid sequences of SEQ ID NOs: 7 to 12 and having 70% or more, 75% or more, 80% or more, 85% or more, 90% or more, 91% or more, 92% or more, 93% or more, 94% or more, 95% or more, 96% or more, 97% or more, 98% or more, or 99% or more identity to the amino acid sequence of SEQ ID NO: 21, or an amino acid comprising the amino acid sequences of SEQ ID NOs: 7 to 12 and having deletion, substitution (e.g., conservative substitution), insertion and/or addition of 1 to 3 amino acids in the amino acid sequence of SEQ ID: 21.

The CAR may be multimerized, such as dimerized. For example, when the CAR is dimerized via a disulfide bond, cysteine is preferably inserted into a spacer domain and/or a transmembrane domain of the CAR.

B-2. Recombinant TCR

The recombinant TCR may be a heterodimer composed of α and β chains or may be a heterodimer composed of γ and δ chains. Each chain has a variable domain (a V domain) and a stationary domain (a C domain), wherein the V domain has three CDRs, i.e., CDR1, CDR2, and CDR3, and is designed such that the three CDR sequences can recognize or bind to an antigen.

The recombinant TCR can recognize (or bind to) any antigen. In other words, the recombinant TCR can have a variable domain capable of recognizing an antigen. Examples of the antigen may be the same as those mentioned in B-1-1. In one embodiment, the antigen is preferably a tumor antigen and is preferably gp100, MART-1, CEA, MAGE-A1, MAGE-A3, MAGE-A4, WT1, or NY-ESO-1. The antigen is preferably human leukemic cell antigen A (HLA-A)-restrictive, and is more preferably HLA-A*02-restrictive (e.g., HLA-A*02:01-restrictive, HLA-A*02:06-restrictive), or HLA-A*24-restrictive (e.g., HLA-A*24:02-restrictive). The recombinant TCR may be HLA-A*02-restrictive gp100-specific TCR, HLA-A*02-restrictive MART-1-specific TCR, HLA-A*02-restrictive MAGE-A3-specific TCR, HLA-A*02-restrictive NY-ESO-1-specific TCR, HLA-A*24-restrictive MAGE-A4-specific TCR, or HLA-A*24-restrictive WT1-specific TCR. The TCR capable of recognizing or binding to NY-ESO-1 is referred to as "NY-ESO-1 TCR." The T cell modified to express NY-ESO-1 TCR is referred to as "NY-ESO-1 TCR-T."

The equilibrium dissociation constant ($K_d$) that represents binding affinity of recombinant TCR to an antigen can be, for example, 100 μM or less, 10 μM or less, 1 μM or less, 500 nM or less, or 100 nM or less, and can be, for example, 0.1 pM or more, 0.5 pM or more, or 1 pM or more. The equilibrium dissociation constant can be determined, for example, by the SPR measurement or Biacore (trademark) analysis.

B-3. Immune Cell Modified to Express CAR or Recombinant TCR

The immune cell modified to express CAR (e.g., CAR-T cells and CAR-NK cells) is preferably an immune cell containing a nucleic acid for CAR expression (containing a nucleic acid construct). Similarly, the immune cell (e.g., TCR-T cells) modified to express a recombinant TCR is preferably an immune cell containing a nucleic acid for recombinant TCR expression (including a nucleic acid construct).

B-3-1. CAR or Recombinant Nucleic Acid for TCR Expression

CAR or a nucleic acid for recombinant TCR expression preferably comprises a promoter in addition to a nucleic acid encoding CAR or recombinant TCR. Examples of the promoter include, but are not limited to, pol II promoters, such as CMV promoter, EF1 promoter, SV40 promoter, and MSCV promoter; tryptophan promoters, such as trc and tac; lac promoter; T7 promoter; T5 promoter; T3 promoter; SP6 promoter; arabinose-inducible promoter; cold shock promoter; tetracycline-inducible promoter; ubiquitin promoter; and the like.

The CAR or nucleic acid for recombinant TCR expression may further contain one or more other elements. Examples of such other elements include, but are not limited to, replication starting points, enhancer sequences, terminator sequences, reporter protein coding sequences, and drug-resistant gene coding sequences.

The nucleic acid for recombinant TCR expression preferably comprises a sequence that inhibits the expression of endogenous TCR (e.g., siRNAs against endogenous TCR α chain and β chain), as described, for example, in WO2008/153029 (which is incorporated herein in its entirety by reference).

The CAR or nucleic acid for recombinant TCR expression may be in the form of a vector. The vector may be a non-viral vector but is preferably a viral vector.

Examples of the virus vector include, but are not limited to, retrovirus vectors (including oncoretrovirus vectors, lentiviral vectors, and pseudotype vectors), adeno virus vectors, adeno-associated virus (AAV) vectors, simian virus vectors, vaccinia virus vectors, or Sendai virus vectors, Epstein-Barr virus (EBV) vectors, and HSV vectors. A virus vector that lacks self-reproduction ability so that it does not reproduce itself within infected cells can also be used as the virus vector.

B-3-2. Immune Cell

The immune cell is not particularly limited as long as it can be modified to express CAR or recombinant TCR. Examples of the immune cell include, but are not limited to, monocytes, macrophages, T cells, B cells, and NK cells. In one embodiment, the immune cell is preferably a T cell or an NK cell. Examples of T cells include, but are not limited to, CD4-positive T cells (e.g., helper T cells), CD8-positive T cells (e.g., cytotoxic T cells), natural killer T cells, regulatory

T cells (Treg), naive T cells, effector T cells, memory T cells, tumor-infiltrating lymphocytes (TIL), αβ T cells, and γδ T cells. The immune cells can typically be a cell population. The cell population can be, for example, a cell population containing peripheral blood mononuclear cells (PBMC). Although the immune cells may be differentiated from hematopoietic stem cells, the immune cells are preferably cells derived from the living body, such as cells collected from a living body (e.g., peripheral blood, cord blood, bone marrow, lymph nodes, and spleen), or cells collected from a living body and subjected to expansion culture. The immune cell may be autologous immune cells (immune cells of a subject being administered with pharmaceutical composition B) or allogeneic immune cells. When an allogeneic immune cell is used as the immune cell, the HLA type is preferably the same.

In one embodiment, the immune cell may be stimulated with soluble or membrane-bound anti-CD3 antibody (e.g., OKT3 or mOKT3) and/or APC (e.g., artificial APC) before the transduction. APC may express a membrane-type anti-CD3 monoclonal antibody.

B-3-3. Transduction of Nucleic Acid for CAR or Recombinant TCR Expression into Immune Cell

Examples of the method for transducing CAR or a nucleic acid for recombinant TCR expression into the immune cell include, but are not limited to, the viral infection method, the calcium phosphate method, the lipofection method, the microinjection method, and the electroporation method.

B-3-4. Non-limiting Examples of Modified Immune Cells

Among the modified immune cells, examples of CAR-T cells include, but are not limited to, T cells modified to express CD19 CAR (e.g., Yescarta (axicabtagene ciloleucel) produced by Gilead, Kymriah (tisagenlecleucel) produced by Novartis, Breyanzi (lisocabtagene maraleucel) produced by Bristol-Myers Squib, TBI-1501 produced by Takara Bio Inc., and UCART19/ALLO-501 produced by Nihon Servier), T cells modified to express BCMA CAR (e.g., bb2121, ide-cel, produced by Celgene Corporation and JNJ-68284528 produced by Janssen Pharmaceutical K.K.), T cells modified to express CD98 CAR (e.g., T cells modified to express CAR described in US Patent Application Publication No. 2018/0230212, which is incorporated herein in its entirety by reference), and T cells modified to express activated integrin β7 CAR (e.g., US Patent Application Publication No. 2018/0230216, which is incorporated herein in its entirety by reference). Examples of TCR-T cells include, but are not limited to, NY-ESO-1-specific TCR-T cells (e.g., NY-ESO-1 TCR-T Triple Knockout TCR (NYCE) produced by Tmunity and TBI-1301 produced by Takara Bio Co., Ltd.), MAGE-A4-specific TCR-T cells (e.g., ADP-A2M4 SPEAR T-cells produced by Adaptimmune), and MAGE-A3 and/or MAGE-A6-specific TCR-T cells (e.g., KITE-718 produced by Kite/Gilead Sciences). In one embodiment, the modified immune cell is preferably a T cell modified to express CAR that specifically recognizes CD19, a T cell modified to express CAR that specifically recognizes integrin β7 (e.g., activated integrin β7), an NK cell modified to express CAR that specifically recognizes CD19, or a T cell modified to express recombinant TCR that specifically recognizes NY-ESO-1.

B-4. Other Components

Pharmaceutical composition B preferably contains a pharmaceutically acceptable excipient as another component. Examples of pharmaceutically acceptable excipients include the same excipients as mentioned in A-2. In one embodiment, the pharmaceutically acceptable excipient is at least one member selected from the group consisting of carriers (e.g., sterile water and physiological saline), stabilizers, antioxidants (e.g., ascorbic acid), buffers (e.g., organic acids such as phosphoric acid and citric acid), preservatives, surfactants (e.g., polyethylene glycol and Tween), chelating agents (e.g., EDTA), sugars or sugar alcohols (e.g., glucose, mannose, sucrose, and mannitol), amino acids (e.g., glycine, glutamine, asparagine, arginine, and lysine), polypeptides or proteins (e.g., serum albumin, gelatin, and immunoglobulin), and adjuvants (e.g., adjuvants such as aluminum hydroxide).

B-5. Administration

The lower limit of the dose of the modified immune cell can be appropriately selected according to the purpose of administration, the subject being administered with the modified immune cell, the condition of the subject, etc. For example, the lower limit of the daily dose in terms of viable cell count per kilogram of body weight of the subject can be $1 \times 10^2$ cells, $5 \times 10^2$ cells, $1 \times 10^3$ cells, $2 \times 10^3$ cells, $3 \times 10^3$ cells, $4 \times 10^3$ cells, $5 \times 10^3$ cells, $6 \times 10^3$ cells, $7 \times 10^3$ cells, $8 \times 10^3$ cells, $9 \times 10^3$ cells, $1 \times 10^4$ cells, $2 \times 10^4$ cells, $3 \times 10^4$ cells, $4 \times 10^4$ cells, $5 \times 10^4$ cells, $6 \times 10^4$ cells, $7 \times 10^4$ cells, $8 \times 10^4$ cells, $9 \times 10^4$ cells, or $1 \times 10^5$ cells. The upper limit of the daily dose can also be appropriately selected as with the lower limit. For example, the upper limit of the daily dose in terms of viable cell count per kilogram of body weight of the subject can be $1 \times 10^9$ cells, $5 \times 10^8$ cells, $1 \times 10^8$ cells, $5 \times 10^7$ cells, $1 \times 10^7$ cells, $5 \times 10^6$ cells, or $1 \times 10^6$ cells, per kilogram of body weight of the subject. For example, the daily dose per kilogram of body

weight of the subject in terms of viable cell count can be 1 × $10^3$ cells to 1 × $10^8$ cells, 5 × $10^3$ cells to 5 × $10^7$ cells, or 1 × $10^4$ cells to 1 × $10^7$ cells. The pharmaceutical composition can be administered in two or more divided doses (e.g., 2 or 3 times) so that the daily dose is within the range described above, or may be administered in divided doses over a period of two days. The dose of the modified immune cell may be less than a dose if administered alone or an amount that is not effective if used alone. Even when the modified immune cell is administered in such an amount, the modified immune cell can provide excellent efficacy due to the administration in combination with a tolinapant drug.

Pharmaceutical composition B can be administered at any frequency. For example, pharmaceutical composition B can be administered, once, twice, or three times a day, once every two days, once every three days, once every four days, once every five days, once every six days, once a week, once every two weeks, once every three weeks, or once every four weeks in frequency. Pharmaceutical composition B can be continued to be administered at a frequency described above (e.g., once a day) for a predetermined period of time (e.g., 1, 2, 3, or 4 weeks), and then the administration may be stopped for a predetermined period of time (e.g., 1, 2, 3 or 4 weeks), and a cycle of continued administration and cessation can be repeated.

In one embodiment, the administration of pharmaceutical composition B (or modified immune cell) preferably comprises the steps of:

(1) administering pharmaceutical composition B (or modified immune cell);
(2) administering pharmaceutical composition B (or modified immune cell) that is produced by using an immune cell obtained from the subject having been administered with pharmaceutical composition B (or modified immune cell); and
(3) repeating step (2), if necessary.

Pharmaceutical composition B may be administered before administration of a tolinapant drug, such as immediately before the administration, or 1 minute before, 2 minutes before, 3 minutes before, 4 minutes before, 5 minutes before, 10 minutes before, 20 minutes before, 30 minutes before, 1 hour before, 2 hours before, 3 hours before, 4 hours before, 5 hours before, 6 hours before, 7 hours before, 8 hours before, 9 hours before, 10 hours before, 11 hours before, 12 hours before, 18 hours before, 24 hours before, 2 days before, 3 days before, 4 days before, 5 days before, 6 days before, or 1 week before the administration. Pharmaceutical composition B may be administered after administration of a tolinapant drug, such as immediately after the administration, or 1 minute after, 2 minutes after, 3 minutes after, 4 minutes after, 5 minutes after, 10 minutes after, 20 minutes after, 30 minutes after, 1 hour after, 2 hours after, 3 hours after, 4 hours after, 5 hours after, 6 hours after, 7 hours after, 8 hours after, 9 hours after, 10 hours after, 11 hours after, 12 hours after, 18 hours after, 24 hours after, 2 days after, 3 days after, 4 days after, 5 days after, 6 days after, or 1 week after the administration. Pharmaceutical composition B may also be administered, for example, after administration of a tolinapant drug and when the tumor marker level is higher than the baseline level.

During the period between the administration of pharmaceutical composition B and the administration of a tolinapant drug or during the period after the administration of pharmaceutical composition B and before the administration of the next pharmaceutical composition B, for example, other chemotherapy (administration of one or more other drugs) or non-chemotherapy (e.g., radiation therapy, photodynamic therapy, surgery, and dietary restrictions) may be performed. Examples of other drugs include, but are not limited to, alkylating agents, DNA methylation inhibitors, antimetabolites (e.g., pyrimidine antimetabolites, purine antimetabolites, and folate antimetabolites).

The subject being administered with pharmaceutical composition B can be, for example, the same as the subject being administered with pharmaceutical composition A. The subject being administered with pharmaceutical composition B may be a subject being or having been administered with a tolinapant drug. The subject being administered with pharmaceutical composition B is preferably a subject (or a new patient or a relapse patient) in need of prevention and/or treatment of an IAP-related disease, in particular, cancer.

The formulation, production method, and administration method for pharmaceutical composition B can be designed based on, for example, the description in WO1993/019163 (which is incorporated herein in its entirety by reference).

B-6. Pharmaceutical Application

As long as pharmaceutical composition B is used in such a mode that its combined use with pharmaceutical composition A can increase the efficacy (therapeutic effect in gene-modified immunotherapy) thereof, the application is not particularly limited. The target disease of pharmaceutical composition B is, for example, a disease to which gene-modified immunotherapy is applicable, typically cancer. Examples of the cancer include, but are not limited to, the same as the cancers mentioned as examples in pharmaceutical composition A.

C. Kit

The kit of the present disclosure preferably comprises a first agent and a second agent,

the first agent comprising a compound represented by formula (1), a pharmaceutically acceptable salt thereof, or a pharmaceutically acceptable solvate thereof, and

the second agent comprising an immune cell modified to express a chimeric antigen receptor or a recombinant T-cell receptor.

The first agent preferably comprises pharmaceutical composition A. The second agent preferably comprises pharmaceutical composition B. The first agent and the second agent may be in the same formulation and administered simultaneously, or the first and second agents are in different formulations and are administered simultaneously, separately, or sequentially.

The various features (properties, structures, functions, etc.) described above in each of the embodiments can be combined in any way to specify the subject matter included in the present disclosure. The present disclosure is not limited to the embodiments described above but includes everything that a person skilled in the art could recognize based on the disclosure in the present specification.

Examples

The subject matter included in the present disclosure is described in more detail below. However, the subject matter is not limited to the Examples described below.

1. Test Materials and Test Method

1.1 Test Cell

1.1.1 Test Cell

1-1) Cell name: CD19 CAR-T (produced by Takara Bio Inc.)

1-2) Origin: human peripheral blood-derived T cell

1-3) Source: Accucell and others

Characteristics: a test cell prepared in the following manner. Commercially available human PMBC was stimulated with anti-CD antibody (clone: OKT3) and the resulting cells were allowed to grow in a medium containing IL-2 (250 U/mL) to induce T cells. A sequence comprising a leader sequence, a single-chain variable fragment (scFv) sequence comprising heavy-chain and light-chain variable regions of anti-human CD19 antibody (clone: FMC63) linked together, a linker sequence, a sequence comprising an extracellular region, a transmembrane region, and an intracellular region of CD28 molecule, and an intracellular region of CD3ζ molecule linked was introduced into the T cells using a retroviral vector, and the resulting cells were grown and cultured and then cryopreserved to obtain a test cell.

2-1) Cell name: activated integrin β7 CAR-T cell

2-2) Origin: human peripheral blood-derived T cell

2-3) Source: Accucell and others

Characteristics: a test cell prepared in the following manner. Commercially available human PMBC was stimulated with anti-CD antibody (clone: OKT3) and anti-CD28 antibody (clone: 28.2), and the resulting cells were allowed to grow in a medium containing IL-2 (100 U/mL) to induce T cells. A sequence comprising a single-chain variable fragment (scFv) sequence comprising heavy-chain and light-chain variable regions of anti-human activated integrin β7 antibody (clone: MMG49) linked together, a leader sequence, a linker sequence, a sequence comprising an extracellular region, a transmembrane region, and an intracellular region of CD28 molecule, and an intracellular region of CD3ζ molecule linked was introduced into the T cells using a retroviral vector, and the resulting cells were grown and cultured and then cryopreserved to obtain a test cell.

3-1) Cell name: HLA-A*02:01-restrictive NY-ESO-1 TCR-T (referred to below as "NY-ESO-1 TCR-T")

3-2) Origin: human peripheral blood-derived T cell

3-3) Source: Accucell and others

Characteristics: a test cell prepared in the following manner. Commercially available human PMBC was stimulated with anti-CD antibody (clone: OKT3) and anti-CD28 antibody (clone: 28.2), and the resulting cells were allowed to grow in a medium containing IL-2 (100 U/mL) to induce T cells. The T cells were infected with a retroviral vector MS3II-NYESO1-siTCR (Patent No. 5969468) capable of expressing T-cell receptor (TCR) α chain and β chain specifically recognizing HLA-A*02:01-restrictive NY-ESO-1 and siRNA interfering with endogenous TCR α chain and β chain genes to thereby obtain genetically modified T cells (Patent No. 5271901 and Patent No. 5828861). The obtained T cells were then cryopreserved to obtain a test cell.

4-1) Cell lines name: CD19 CAR KHYG-1

4-2) Origin: human NK-like cell
4-3) Source: JCRB
After obtaining the cell, a CD19 CAR expression line was established.
4-4) Culture conditions: a RPMI 1640 culture medium containing 10% inactivated (treated at 56°C for 30 min) fetal bovine serum, the additive described below, and cytokine; $CO_2$ incubator (37°C, 5% $CO_2$)

a) Culture fluid: RPMI 1640

i) Source: Nacalai Tesque Inc.

b) Serum: fetal bovine serum

i) Concentration: 10%
ii) Source: Sigma-Aldrich

c) Additive: penicillin-streptomycin mixed solution

i) Concentrations: 100 units/mL penicillin, 100 ug/mL streptomycin
ii) Source: Nacalai Tesque Inc.

d) Cytokine: recombinant human IL-2

i) Concentration: 100 U/mL
ii) Source: PeproTech

Characteristics: a test cell prepared in the following manner. After a human NK-like cell line KHYG-1 was transfected using a CD19 CAR retrovirus vector and the resulting cells were allowed to grow, positive cells were sorted by FCM and subjected to expansion culture again, and then cryopreserved to obtain a test cell.

1.1.2 Control Cell

1-1) Cell line name: control T cell (human T cells)
1-2) Origin: human peripheral blood PBMC
1-3) Source: Accucell and others
Characteristics: a test cell obtained in the following manner. Commercially available human PMBC was stimulated with anti-CD antibody (clone: OKT3) and anti-CD28 antibody (clone: 28.2), and the resulting cells were grown and cultured in a medium containing IL-2 (100 U/mL), and then cryopreserved to obtain a test cell.
2-1) Cell line name: KHYG-1
2-2) Origin: human NK-like cell
2-3) Source: JCRB
2-4) Culture conditions: a RPMI 1640 culture medium containing 10% inactivated (treated at 56°C for 30 min) fetal bovine serum, the additive described below, and cytokine; $CO_2$ incubator (37°C, 5% $CO_2$)

a) Culture fluid: RPMI1640

i) Source: Nacalai Tesque, Inc.

b) Serum: fetal calf serum

i) Concentration: 10%
ii) Source: Sigma-Aldrich

c) Additive: penicillin-streptomycin mixed solution

i) Concentrations: 100 units/mL penicillin, 100 ug/mL streptomycin
ii) Source: Nacalai Tesque, Inc.

d) Cytokine: recombinant human IL-2

i) Concentration: 100 U/mL
ii) Source: PeproTech

1.1.3 Preparation of Cell Diluent

To prepare a cell diluent, 47% bicarbon infusion, 20% low molecular dextran injection, 28% human serum albumin 25%, and 5% dimethyl sulfoxide were used. After mixing all solutions, the resulting mixture was stored in a deep freezer (setting temperature: -80°C) until use.

1.1.4 Preparation of Test Cell Administration Solution and Control Cell Administration Solution

1) After cryopreserved CD19 CAR-T was rapidly thawed in a bath with a constant temperature of 37°C, the cells were suspended in cell diluent and centrifuged (800 × g, 5 min, 4°C) to remove a surfactant. After the cells were resuspended in cell diluent, a portion of the suspension was taken and dead cells were stained with AOPI Staining Solution. The number of viable cells was counted using a Cellometer Auto 2000 Cell Viability Counter blood cell measuring device (produced by Nexcelom Bioscience). Based on the measurement results of the viable cell count of the cells, the cells were adjusted using cell diluent according to the group constitution so that the cell solution could be administered at a dose of 0.1 mL/body weight. The cell suspension after the preparation was stored in ice water until administration.
2) After cryopreserved activated integrin β7 CAR-T was rapidly thawed in a bath with a constant temperature of 37°C, the cells were suspended in cell diluent and centrifuged (800 × g, 5 min, 4°C) to remove a surfactant. After the cells were resuspended in cell diluent, a portion of the suspension was taken and dead cells were stained with AOPI Staining Solution. The number of the viable cells was counted using a blood cell measuring device. Based on the measurement results of the viable cell count of the cells, the cells were adjusted using cell diluent according to the group constitution so that the cell suspension could be administered at a dose of 0.1 mL/kg body weight. The cell suspension after preparation was stored in ice water until administration.
3) After cryopreserved Control-T was rapidly thawed in a bath with a constant temperature of 37°C, the cells were suspended in cell diluent and centrifuged (800 × g, 5 min, 4°C) to remove a surfactant. After the cells were resuspended in cell diluent, a portion of the suspension was taken and dead cells were stained with AOPI Staining Solution. The number of the viable cells was counted using a blood cell measuring device. Based on the measurement results of the viable cell count of the cells, the cells were adjusted using cell diluent so that the cell suspension could be administered at a dose of 0.1 mL/kg body. The cell suspension after preparation was stored in ice water until administration.
4) After cryopreserved NY-ESO-1 TCRI-T was rapidly thawed in a bath with a constant temperature of 37°C, the cells were suspended in cell diluent and centrifuged (800 × g, 5 min, 4°C) to remove a surfactant. After the cells were resuspended in cell diluent, a portion of the suspension was taken and dead cells were stained with AOPI Staining Solution. The number of the viable cells was counted using a blood cell measuring device. Based on the measurement results of viable cell count of the cells, the cells were adjusted using cell diluent according to the group constitution so that the cell suspension could be administered at a dose of 0.1 mL/body. The cell suspension after preparation was stored in ice water until administration.
5) After cryopreserved CD19 CAR-KHYG-1 and KHYG-1 were rapidly thawed in a bath with a constant temperature of 37°C, the cells were suspended in culture media and centrifuged (150 × g, 5 min, 20°C) to remove surfactants. After the cells were resuspended in the media, the entire amount of the cells was seeded into flasks and cultured at 37°C and 5% $CO_2$. After the culture was started, cell morphology and proliferation were observed under a microscope. The cells were cultured until the cells were grown to subconfluency. The subconfluent cell suspension was passaged into culture flasks containing a new culture medium. The same operation was then continued to perform expansion culture. The cells necessary for administration were maintained during the test period.

On each administration day, each cell suspension cultured until the cells were grown to subconfluency was collected in a required amount and washed 3 times with D-PBS (-) by centrifugation. The cells were then resuspended in serum-free RPMI1640. After re-suspension, a portion of the cell suspension was taken and dead cells were stained with AOPI Staining Solution. The number of the viable cells was counted using a blood cell measuring device. Based on the measurement results of viable cell count of the cells, the cells were adjusted using cell diluent according to the group constitution so that the cell suspension could be administered at a dose of 0.1 mL/body. The administration solution

after preparation was stored in ice water until administration.

1.2 Test Substance

1.2.1 Test Substance

Table 1

| Test substance name | Source/manufacturer |
|---|---|
| Tolinapant | Astex Pharmaceuticals |
| Birinapant | Medchemexpress |
| LCL161 | Medchemexpress |
| AT406 | MedKoo Biosciences |
| Tolinapant: (+)-L-lactate of a compound of formula (1) is represented only in the Examples.<br>Storage conditions: refrigerated, shielded from light | |

1.2.2 Preparation of Media

As the basic medium, distilled water was used for tolinapant, captisol was used for birinapant, sodium acetate was used for LCL161, and hypromellose was used for AT406.

For captisol, the required amount of captisol was weighed and then dissolved in distilled water and adjusted to prepare a 12.5% captisol solution, which was used as a solvent for birinapant.

For sodium acetate, 0.1 M hydrochloric acid was added to a 3M sodium acetate buffer (pH of 5.2) to achieve a pH of 4.3 to 4.6, and the resulting mixture was used as a solvent for LCL161.

For hypromellose, the required amount of hypromellose was weighed and then dissolved in PBS to prepare a 1% hypromellose solution. After dissolution and autoclaving, Tween 80 in an amount equivalent to 0.1% was added and dissolved, and the resulting mixture was used as a solvent for AT406.

All of the media were stored in a low-temperature room (setting temperature: 4°C) until drug preparation.

1.2.3 Preparation of Tolinapant Administration Solution

Tolinapant was prepared so that tolinapant could be administered at a dose of 16 mg/kg, based on published reports (Reference 1: Mol Cancer Ther. 2018 July; 17(7): 1381-1391; and Reference 2: Oncoimmunology, 2020 January 9; 9(1): 1710398). Tolinapant was weighed to an amount required to achieve a concentration of 1.6 mg/mL and then dissolved in distilled water to obtain a colorless transparent solution. The resulting administration solution was dispensed into sterile tubes and cryopreserved under light-shielded conditions until administration.

1.2.4 Preparation of Birinapant Administration Solution

Birinapant was prepared so that birinapant could be administered at a dose of 12 mg/kg, based on a published report (Reference 3: Jessica Michie et al., Cancer Immunol Res. 2019; 7(2): 183-192). Birinapant was weighed to an amount required to achieve 1.2 mg/mL and then suspended in captisol prepared in advance. The suspension was prepared by grinding the resulting mixture to form a uniform fine suspension using an agate mortar. The administration solution was dispensed into sterile tubes and stored in low-temperature chamber (setting temperature: 4°C) under light-shielded conditions until administration.

1.2.5 Preparation of LCL161 Administration Solution

LCL161 was prepared so that LCL161 could be administered at a dose of 30 mg/kg, based on a published report (Reference 4: Peter J Houghton et al., Pediatr Blood Cancer. 2012; 58(4): 636-639). LCL161 was weighted to an amount required to achieve a concentration of 3 mg/mL and then dissolved in a sodium acetate solution prepared in advance to form a colorless transparent solution. The resulting administration solution was dispensed into sterile tubes and stored in a low-temperature chamber (setting temperature: 4°C) under light-shielded conditions until administration.

1.2.6 Preparation of AT406 Administration Solution

AT406 was prepared so that AT406 could be administered at a dose of 100 mg/kg, based on a published document (Reference 5: Melissa K Brunckhorst et al., Cancer Biology & Therapy. 2012; 13(9): 804-811). First, a 200 mg/mL DMSO solution of AT406 was prepared. Subsequently, AT406 was weighed to an amount required to achieve 10 mg/mL and suspended in a hypromellose solution prepared in advance. The suspension was prepared by grinding the mixture to a uniform fine suspension using an agate mortar. The administration solution was dispensed into sterile tubes and stored in low-temperature chamber (set temperature: 4°C) under light-shielded conditions until administration.

1.3 Test System
1.3.1 Cell Used
1.3.1.1 NALM6-luc2
1) Cell line name: NALM6-luc2
2) Origin: acute lymphoblastic leukemia
3) Source: ATCC CRL-3273

After obtaining ATCC CRL-3273, a luc2 cell line was established.
4) Culture conditions: RPMI1640 medium containing 10% inactivated (treated at 56°C for 30 min) fetal bovine serum 10%; $CO_2$ incubator (37°C, 5% $CO_2$)

a) Culture fluid: RPMI1640

i) Source: Nacalai Tesque, Inc.

b) Serum: fetal bovine serum

i) Concentration: 10%
ii) Source: Nichirei Biosciences Inc.

c) Additive: penicillin-streptomycin mixed solution

i) Concentrations: 100 units/mL of penicillin, 100 μg/mL of streptomycin
ii) Source: Nacalai Tesque, Inc.

1.3.1.2 MM.1S-luc2

1) Cell line name: MM. 1S
2) Origin: multiple myeloma
3) Source: ATCC (After obtaining the cell, a luc2 cell line was established.)
4) Culture conditions: a RPMI1640 medium containing 10% inactivated (treated at 56°C for 30 min) fetal bovine serum; $CO_2$ incubator (37°C, 5% $CO_2$)

a) Culture fluid: RPMI1640

i) Source: Nacalai Tesque, Inc.

b) Serum: fetal bovine serum

i) Concentration: 10%
ii) Source: Sigma-Aldrich

c) Additive: penicillin-streptomycin mixed solution

i) Concentrations: 100 units/mL of penicillin, 100 μg/mL of streptomycin
ii) Source: Nacalai Tesque, Inc.

1.3.1.3 A375

1) Cell strain name: A375
2) Origin: malignant melanoma
3) Source: ATCC CRL-1619
4) Culture conditions: a DMEM medium containing 10% inactivated (treated at 56°C, 30 min) fetal bovine serum; $CO_2$ incubator (37°C, 5% $CO_2$)

a) Culture medium: DMEM

i) Source: Nacalai Tesque, Inc.

b) Serum: fetal bovine serum

i) Concentration: 10%
ii) Source: Sigma-Aldrich

c) Additive: penicillin-streptomycin mixed solution

i) Concentration: 100 units/mL of penicillin, 100 μg/mL of streptomycin
ii) Source: Nacalai Tesque, Inc.

1.3.1.4 NALM6

1) Cell line name: NALM6
2) Origin: acute lymphoblastic leukemia
3) Source: ATCC CRL-3273
4) Culture conditions: a RPMI1640 medium containing 10% inactivated (treated at 56°C for 30 min) fetal bovine serum; $CO_2$ incubator (37°C, 5% $CO_2$)

a) Culture fluid: RPMI1640

i) Source: Nacalai Tesque, Inc.

b) Serum: fetal bovine serum

i) Concentration: 10%
ii) Source: Sigma-Aldrich

c) Additive: penicillin-streptomycin mixed solution

i) Concentration: 100 units/mL of penicillin, 100 μg/mL of streptomycin
ii) Source: Nacalai Tesque, Inc.

1.3.2 Animal

1) Species/strain: mouse/NOD/Shi-scid, IL-2RγKO Jic (NOG)
2) Microbiological grade: specific-pathogen-free (SPF)
3) Source: In-Vivo Science Inc.
4) Age in weeks (at the time of receipt): 6 weeks old
5) Breeding conditions

The animals were bred in an environment set to the following conditions:

a) temperature: 23 ± 3°C
b) humidity: 55 ± 15%
c) illumination: lighting time: 8:00 a.m. to 8:00 p.m.
light-out time: 8:00 p.m. to 8:00 a.m.
d) food and water: freely available, CL-2 (solid feed, radiation-sterilized, produced by CLEA Japan, Inc.), autoclaved tap water

1.3.3 Group Constitution

1.3.3.1 Combination of IAP Antagonist and CD19 CAR-T

The group constitution in each test is as follows.

Table 2: Birnapant combination test

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | Birinapant | 12 mg/kg, q3-4d, ip | - | - | 5 | ♀ |
| 3 | CD19 CAR-T | 1×$10^5$ cells/body, iv | - | - | 5 | ♀ |
| 4 | CD19 CAR-T | 1×$10^6$ cells/body, iv | - | - | 5 | ♀ |
| 5 | Birinapant | 12 mg/kg, q3-4d, ip | CD19 CAR-T | 1×$10^5$ cells/body, iv | 5 | ♀ |

Table 3: Tolinapante combination test

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | Tolinapant | 16 mg/kg, qd, po | - | - | 5 | ♀ |
| 3 | CD19 CAR-T | 1×$10^5$ cells/body, iv | - | - | 5 | ♀ |
| 4 | CD19 CAR-T | 1×$10^6$ cells/body, iv | - | - | 5 | ♀ |
| 5 | Tolinapant | 16 mg/kg, qd, po | CD19 CAR-T | 1×$10^5$ cells/body, iv | 5 | ♀ |

Table 4: Tolinapante combination test stated with a low tumor volume[a]

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | Tolinapant | 16 mg/kg, qd, po | - | - | 5 | ♀ |
| 3 | CD19 CAR-T | 2×$10^4$ cells/body, iv | - | - | 5 | ♀ |
| 4 | CD19 CAR-T | 1×$10^5$ cells/body, iv | - | - | 5 | ♀ |
| 5 | CD19 CAR-T | 1×$10^6$ | - | - | 5 | ♀ |
| | | cells/body, iv | | | | |

(continued)

| Group | Administration 1 | Dosage and administration for administration 1 | Administration 2 | Dosage and administration for administration 2 | Number n | Male or female |
|---|---|---|---|---|---|---|
| 6 | Tolinapant | 16 mg/kg, qd, po | CD19 CAR-T | $2\times10^4$ cells/body, iv | 5 | ♀ |
| 7 | Tolinapant | 16 mg/kg, qd, po | CD19 CAR-T | $1\times10^5$ cells/body, iv | 5 | ♀ |

[a] "Low tumor volume" refers to an evaluation test on a combination of drugs starting with a tumor engraftment volume that is approximately one-tenth the volume of the conventional evaluation system (luminescence amount of approximately $2\times10^6$ photons/sec).

Table 5: Combination test of LCL161 and AT406

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | LCL161 | 30 mg/kg, qw, po | - | - | 5 | ♀ |
| 3 | AT406 | 100 mg/kg, qd, po | - | - | 5 | ♀ |
| 4 | CD19 CAR-T | $1\times10^5$ cells/body, iv | - | - | 5 | ♀ |
| 5 | CD19 CAR-T | $1\times10^6$ cells/body, iv | - | - | 5 | ♀ |
| 6 | LCL161 | 30 mg/kg, qw, po | CD19 CAR-T | $1\times10^5$ cells/body, iv | 5 | ♀ |
| 7 | AT406 | 100 mg/kg, qd, po | CD19 CAR-T | $1\times10^5$ cells/body, iv | 5 | ♀ |

1.3.3.2 Combination of IAP Antagonist and Activated Integrin β7 CAR-T (aITGB7 CAR-T)

The group constitution in this test is as follows. Table 6: Tolinapant combination test

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | - | - | Tolinapant | 16 mg/kg, pd, po | 5 | ♀ |
| 3 | Control-T | $3\times10^4$ cells/body, iv | - | - | 5 | ♀ |
| 4 | Control-T | $1\times10^5$ cells/body, iv | - | - | 5 | ♀ |
| 5 | aITGB7 CAR-T | $3\times10^4$ cells/body, iv | - | - | 5 | ♀ |
| 6 | aITGB7 CAR-T | $1\times10^5$ cells/body, iv | - | - | 5 | ♀ |
| 7 | Control-T | $3\times10^4$ cells/body, iv | Tolinapant | 16 mg/kg, pd, po | 5 | ♀ |
| 8 | Control-T | $1\times10^5$ cells/body, iv | Tolinapant | 16 mg/kg, pd, po | 5 | ♀ |
| 9 | aITGB7 CAR-T | $3\times10^4$ cells/body, iv | Tolinapant | 16 mg/kg, pd, po | 5 | ♀ |
| 10 | aITGB7 CAR-T | $1\times10^5$ cells/body, iv | Tolinapant | 16 mg/kg, pd, po | 5 | ♀ |

1.3.3.3 Combination of IAP Antagonist and NY-ESO-1-TCR-T

The group constitution in this test is as follows.

Table 7: Tolinapant combination test

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | Tolinapant | 16 mg/kg, pd, po | - | - | 5 | ♀ |
| 3 | - | - | NY-ESO-1 TCR-T | $3\times10^6$ cells/body, iv | 5 | ♀ |
| 4 | - | - | NY-ESO-1 TCR-T | $1\times10^7$ cells/body, iv | 5 | ♀ |
| 5 | Tolinapant | 16 mg/kg, pd, po | NY-ESO-1 TCR-T | $3\times10^6$ cells/body, iv | 5 | ♀ |
| 6 | Tolinapant | 16 mg/kg, pd, po | NY-ESO-1 TCR-T | $1\times10^7$ cells/body, iv | 5 | ♀ |

1.3.3.4 Combination of IAP Antagonist and CD19 CAR-KHYG-1

The group constitution in this test is as follows.

Table 8: Tolinapant combination test

| Group | Administration 1 | Dosage and administration for administration 1 | Administration 2 | Dosage and administration for administration 2 | Number n | Male or female |
|---|---|---|---|---|---|---|
| 1 | - | - | - | - | 5 | ♀ |
| 2 | - | - | Tolinapant | 16 mg/kg, pd, po | 5 | ♀ |
| 3 | KHYG-1 | $1\times10^7$ cells/body, it Day 0, 4, 7, 11 | - | - | 5 | ♀ |
| 4 | CD19 CAR-KHYG-1 | $1\times10^7$ cells/body, it Day 0, 4, 7, 11 | - | - | 5 | ♀ |
| 5 | KHYG-1 | $1\times10^7$ cells/body, it Day 0, 4, 7, 11 | Tolinapant | 16 mg/kg, pd, po | 5 | ♀ |
| 6 | CD19 CAR-KHYG-1 | $1\times10^7$ cells/body, it Day 0, 4, 7, 11 | Tolinapant | 16 mg/kg, pd, po | 5 | ♀ |
| it: intratumoral injection | | | | | | |

1.3.4 Rationale for Dosage and Administration Setting

1.3.4.1 Tolinapant

The dosage and administration of tolinapant were set based on the dosage and administration that exhibited efficacy in human breast cancer cell line MDA-MB-231 xenograft mice generally used in IAP antagonist tests (16 mg/kg, daily oral administration) (References 1 and 2). It was also confirmed that this test system NALM6-luc2 showed no efficacy in the same regimen.

1.3.4.2 Birinapant

The dosage and administration of birinant were set based on a paper regarding a combination of CAR-T and birinapant (Reference 3) (12 mg/kg, intraperitoneal administration twice a week). It was also confirmed that this test system NALM6-luc2 showed no efficacy in the same regimen.

1.3.4.3 LCL161

The dosage and administration of LCL161 were set with reference to the lowest dose shown in a published report (30 mg/kg, oral administration twice a week) (Reference 4). It was confirmed that when this test system NALM6-luc2 was orally administered in an amount of 30 mg/kg twice a week, no effects were exhibited and at least half of the mice died. Therefore, the dosage and administration were reduced to once-a-week oral administration (30 mg/kg, oral administration once a week).

1.3.4.4 AT406

Based on the dosage and administration at which efficacy in a human ovarian cancer cell line OVCAR xenograft mouse system and a human breast cancer cell line MDA-MB-231 xenograft mouse system was reported in published documents, the maximum dose without drug cessation (100 mg/kg, daily oral administration) was set (Reference 5; Reference 6: Qian Cai et al. Med Chem. 2011; 54(8): 2714-2726). It was also confirmed that this test system NALM6-luc2 showed no efficacy in the same regimen.

1.3.4.5 CD19 CAR-KHYG-1 and KHYG-1

In a pharmacological test of Ev CAR-KHYG-1 using KHYG-1 in a subcutaneous tumor mouse model of a glioblastoma cell line, the pharmaceutical composition was intratumorally administered at a dose of $1\times10^7$ cells/body twice a week (3 times in total) (Reference 7: Nakazawa T et al., Anticancer Res. 2020; 40(6): 3231-3237). In FOLR1 CAR-KHYG-1 using a gastric cancer cell line, intratumoral administration was performed once a week (twice in total) (Reference 8: Minsung Kim et al., PLoS One. 2018; 13(6): e0198347).

1.4 Test Method

1.4.1 Method for Testing Combination of IAP Antagonist and CD19 CAR-T

6-week-old NOG mice produced by In-Vivo Science Co., Ltd., were obtained, and tumor cells were intravenously transplanted into the mice at 7 weeks of age to prepare tumor-bearing mice. The day before the transplantation, busulfex (BSF) was intraperitoneally administered at 20 mg/kg. During the breeding period, the mice were housed with 5 mice per group and bred in a P2A breeding room set to a temperature of 24°C (actual measurement: 19.7 to 25.0°C), humidity of 50% (actual measurement: 38.6 to 58.5%), and illumination of 12 hours/day (8:00 a.m. to 8:00 p.m.). During the breeding period, the mice were allowed free access to radiation-sterilized solid feed for mice (CL-2, produced by Clea Japan, Inc.) and autoclave-sterilized tap water placed in a water bottle.

CD19 CAR-T cells were intravenously administered once according to the group constitution on the same day as grouping. The IAP antagonist also started to be administered on the same day as grouping and was orally or intravenously administered according to the group constitution table.

The evaluation period was set to 3 to 4 weeks. Grouping and pharmacological evaluation were performed based on tumor luminescence values as determined by IVIS (trademark). Luminescence value measurement by IVIS (trademark) and body weight measurement were performed twice a week.

1.4.2 Method for Testing Combination of IAP Antagonist and Activated Integrin β7 CAR-T

6-week-old NOG mice produced by In-Vivo Science Co., Ltd., were obtained, and tumor cells were intravenously transplanted into the mice at 7 weeks of age to prepare tumor-bearing mice. Each mouse was identified with a number attached to the tail of the mouse. During the breeding period, the mice were housed in groups of 5 mice each and bred in a breeding room at a temperature of 24°C (actual measurement: 19.7 to 25.0°C), humidity of 50% (actual measurement: 38.6 to 58.5%), and illumination of 12 hours/day (8:00 a.m. to 8:00 p.m.). During the breeding period, the mice were allowed free access to radiation-sterilized solid feed for mice (CL-2, produced by Clea Japan, Inc.) and autoclave-sterilized tap water placed in a water bottle.

Control-T cells and activated integrin β7 CAR-T (aITGB7 CAR-T) cells were intravenously administered once according to the group constitution table on the same day as grouping. Tolinapant also started to be administered on the same day as grouping and was orally administered every day according to the group constitution table.

The evaluation period was set to up to day 14 or day 21. Grouping and pharmacological evaluation were performed using tumor luminescence values as determined by IVIS as an indicator. The luminescence value as determined by IVIS and body weight were measured twice a week.

1.4.3 Method for Testing Combination of IAP Antagonist and NY-ESO-1 TCR-T

6-week-old NOG mice produced by In-Vivo Science Inc. were obtained, and tumor cells were subcutaneously implanted at 7 weeks of age to prepare tumor-bearing mice. Each mouse was identified with a number attached to the tail of the mouse. During the breeding period, the mice were housed in groups of 5 mice each and bred in a breeding room at a temperature of 23°C, humidity of 55%, and illumination of 12 hours/day (8:00 a.m. to 8:00 p.m.). During the breeding period, the mice were allowed free access to radiation-sterilized solid feed for mice (CL-2, produced by Clea Japan, Inc.) and autoclave-sterilized tap water placed in a water bottle.

NY-ESO-1 TCR-T cells were intravenously administered once according to the group constitution table on the same day as grouping. Tolinapant also started to be administered on the same day as grouping and was orally administered according to the group constitution table.

The evaluation period was set to 2 weeks, and grouping and pharmacological evaluation were performed based on the results of tumor diameter measurement using a caliper. The tumor diameter and body weight were measured twice a week.

1.4.4 Method for Concomitant Test of IAP Antagonist and CD19 CAR-KHYG-1

6-week-old NOG mice produced by In-Vivo Science Inc. were obtained, and tumor cells were subcutaneously transplanted at 7 weeks of age to prepare tumor-bearing mice. Each mouse was identified with a number attached to the tail of the mouse. During the breeding period, the mice were housed in groups of 5 mice each and bred in a breeding room at a temperature of 24°C (actual measurement: 19.7 to 25.0°C), humidity of 50% (actual measurement: 38.6 to 58.5%), and illumination of 12 hours/day (8:00 a.m. to 8:00 p.m.). During the breeding period, the mice were allowed free access to radially-sterilized solid feed for mice (CL-2, produced by Clea Japan, Inc.,) and autoclave-sterilized tap water placed in a water bottle.

After grouping, CD19 CAR KHYG-1 cells and KHYG-1 cells were intratumorally administered 4 times in total according to the group constitution. Tolinapant also started to be administered on the same day as grouping and was orally administered every day according to the group constitution table.

The evaluation period was set up to day 17. Grouping and pharmacological evaluation were performed using the results of tumor volume measurement with a caliper as an indicator. The tumor volume measurement and body weight measurement were performed twice a week and before each administration.

1.4.1 Preparation of Medium for Culture

1.4.1.1 Preparation of RPMI1640 Medium

50 mL of inactivated (treated at 56°C for 30 min) fetal bovine serum and 5 mL of an antibiotic were added to 500 mL of RPMI 1640 culture medium and the resulting medium was used as a culture medium. On the other hand, for KHYG-1 cells and CD19 CAR-KHYG-1 cells, the medium further containing IL-2 in a final concentration of 100 U/mL was used as a culture medium. Each medium was stored in a low-temperature chamber (setting temperature: 4°C) until use.

1.4.1.2 Preparation of DMEM Medium

50 mL of inactivated (treated at 56°C for 30 min) fetal bovine serum and 5 mL of an antibiotic were added to 500 mL of a DMEM medium. The resulting medium was stored in a low-temperature chamber (setting temperature: 4°C) until use.

1.4.2 Preparation of Cell Suspensions for Transplantation

1.4.2.1 Preparation of NALM6-luc2, MM.1S-luc2, and NALM6 Cell Suspensions for Transplantation

Cell culture was performed using a culture medium referred to below as "medium") in a $CO_2$ incubator (5% $CO_2$, 37°C) . After a cryopreserved cell line was rapidly thawed in a bath with a constant temperature of 37°C, the cells were suspended in the medium and centrifuged (150 × g, 5 min, 20°C) to remove a suspension. The cells were resuspended in the medium and the entire amount of the cells were seeded in a flask and cultured at 37°C in the presence of 5% $CO_2$. After starting the culture, cell morphology and proliferation were observed under a microscope. The cells were cultured until the cells were grown to subconfluency (first passage). The subconfluent cell suspension was passaged into a culture flask containing a new medium (the second passage). After thawing, the cells passaged 3 times or more

were used for transplantation. The volume of the culture fluid to be passaged was 1/10 to 1/3 of the liquid volume of the new culture medium.

The cell suspension cultured to subconfluency was washed 3 times with D-PBS (-) by centrifugation and then resuspended in serum-free RPMI 1640.

For NALM6-luc2, after resuspension, a portion of the cell suspension was taken, dead cells were stained with AOPI Staining Solution, and the number of viable cells was counted with a blood cell measuring device. A cell suspension was prepared by diluting the cells with RPMI 1640 to $1 \times 10^7$ cells/mL, based on the measurement results of viable cell count of the cells. Similarly, in a low tumor volume system, a cell suspension was prepared by diluting the cells with serum-free RPMI 1640 to achieve a concentration of $1 \times 10^6$ cells/mL.

For MM.1S-luc2, after resuspension, a portion of the cell suspension was taken, dead cells were stained with AOPI Staining Solution, and the number of viable cells was counted with a blood cell measuring device. A cell suspension was prepared by diluting the cells with RPMI 1640 to $3 \times 10^7$ cells/mL, based on the measurement results of viable cell count of the cells.

For NALM6, after resuspension, a portion of the cell suspension was taken, dead cells were stained with AOPI Staining Solution, and the number of viable cells was counted with a blood cell measuring device A cell suspension was prepared by diluting the cells with RPMI 1640 to $1 \times 10^8$ cells/mL, based on the measurement results of viable cell count of the cells. The prepared cell suspension was mixed in an equal amount with a matrigel as a scaffold (trademark: Corning, high-concentration matrigel basement membrane matrix) to obtain a final suspension for transplantation ($5 \times 10^7$ cells/mL).

The cell suspensions after the preparation were stored in ice water until transplantation.

1.4.2.2 Preparation of A375 Cell Suspension for Transplantation

Cell culture was performed in a $CO_2$ incubator (5% $CO_2$, 37°C) using a culture medium (referred to below as "culture"). After a cryopreserved A375 cell line was rapidly thawed in a bath with a constant temperature of 37°C, the cells were suspended in medium and centrifuged (150 × g, 5 min, 20°C) to remove a suspension. After the cells were resuspended in the medium, the entire amount of the cells was seeded in a dish and cultured at 37°C in the presence of 5% $CO_2$. After starting the culture, cell morphology and proliferation were observed under a microscope, and the cells were cultured until the cells were grown to subconfluency (first passage). After removing the supernatant from the subconfluent in the dish and washing with D-PBS(-) (produced by Nacalai Tesque, Inc.), trypsin-EDTA-PBS(-) (produced by Nacalai Tesque Inc.) was added and the cells were detached and collected and then centrifuged (4°C, 1200 rpm, 5 min.). The cells were suspended with a DMEM medium and the number of viable cells was counted using AOPI Staining Solution. The cells were seeded at a cell density suitable for the dish size and cultured in a $CO_2$ incubator (the second passage). The cells passaged 3 times or more were used for transplantation.

Using trypsin-EDTA-PBS(-), the cultured cells were detached and collected and then centrifuged (4°C, 1200 rpm, 5 min). After removing the supernatant, D-PBS (-) was added and the cells were resuspended and washed by centrifugation in the same manner as above. This washing operation was further repeated twice. After a portion of the cell suspension was taken and the number of viable cells was counted using AOPI Staining Solution, a cell suspension at $1 \times 10^7$ cells/mL was prepared using D-PBS(-). After the preparation, the cell suspension was stored in ice until transplantation.

1.4.3 Transplantation

1.4.3.1 NALM6-Luc2 Transplantation

After the mice were retained using a retainer, a cell suspension was transplanted at $1 \times 10^6$ cells/0.1 mL/body using a 27G Myjector (1 mL, produced by Terumo Corporation) to the tail vein of each mouse with no anesthesia. In the low tumor volume system, a cell suspension at $1 \times 10^5$ cells/0.1 mL/body was transplanted.

1.4.3.2 MM.1S-luc2 Transplantation

After the mice were retained using a retainer, a cell suspension was transplanted at $3 \times 10^6$ cells/0.1 mL/body to the tail vein of each mouse with no anesthesia using a 27G Myjector (1 mL, produced by Terumo Corporation).

1.4.3.3 A375 Transplantation

In a subcutaneous transplantation group, to facilitate the transplant operation and subsequent tumor growth observation, the transplant site (right axillary area) was shaved using a safety razor by the day before tumor transplantation. To prepare tumor-bearing mice, a cell suspension was transplanted at 1 $\times$ $10^6$ cells/0.1 mL/body into the axillary subcutis of the mice with no anesthesia using an insulin syringe with a 26G needle (1 mL, Terumo Corporation).

1.4.3.4 NALM6 Transplantation

In a subcutaneous transplantation group, to facilitate the transplant operation and subsequent tumor growth observation, the transplant site (right axillary area) was shaved using a safety razor by the day before tumor transplantation. To prepare tumor-bearing mice, a cell suspension was transplanted at 5 $\times$ $10^6$ cells/0.1 mL/body into the axillary subcutis of mice with no anesthesia using an insulin syringe with a 26G needle (1 mL, Terumo Corporation).

1.4.4 Grouping

1.4.4.1 Grouping after Transplantation of NALM6-Luc2

In accordance with the dosing regimen shown in Fig. 6, NALM6-luc2 was transplanted the day after treating the cells with busulfex. On the third day after the transplantation, luminescence values were measured using the IVIS (trademark) Lumina X5 In-Vivo imaging system (produced by PerkinElmer, Co., Ltd.). Using the luminous values as an indicator and using SAS software R9.4 (produced by SAS Institute Japan), the mice were divided into groups of 5 mice each by the stratified random sampling method.

1.4.4.2 Grouping after Transplantation of MM.1S-luc2

According to the dosing regimen shown in Fig. 9, on the seventh day after the transplantation, luminescence values were measured using the IVIS (trademark) Lumina X5 In-Vivo imaging system (produced by PerkinElmer, Co., Ltd.). Using the luminescence values as an indicator and using SAS software R9.4 (produced by SAS Institute Japan), the mice were divided into groups of 5 mice each by the stratified random sampling method. After grouping, the individuals were identified by marking on the tail of each mouse.

1.4.4.3 Grouping after A375 Transplantation

On the eighth day after the transplantation, the long diameter and the short diameter were measured and the tumor volume was calculated. Using the SAS software R9.4 (produced by SAS Institute Japan) and using the tumor size as an indicator, the mice were divided into groups of 5 mice each by the stratified random sampling method. After grouping, the individuals were identified by marking on the tail of each mouse.

1.4.4.4 Grouping after NALM6 Transplantation

On the 21th day after the transplantation, the long diameter and the short diameter were measured and the tumor volume was calculated. Using the SAS software R9.4 (produced by SAS Institute Japan) and using the tumor size as an indicator, the mice were divided into groups of 5 mice each by the stratified random sampling method. After grouping, the individuals were identified by marking on the tail of each mouse.

1.4.5 Administration Method

1) A suspension of CD19 CAR-T cells was administered to the tail vein at the time of grouping. For the dosage, the number of viable CD19 CAR-T cells was counted and a cell suspension prepared according to the group constitution table was administered once at 0.1 mL/body. For the administration, a Myjector syringe with a 27G needle was used.
2) An activated integrin β7 CAR-T (aITGB7 CAR-T) cell suspension and a control-T cell suspension were administered to the tail vein at the time of grouping. For the dosage, the number of viable aITGB7 CAR-T cells was counted a cell suspension prepared according to the group constitution table was administered once at 0.1 mL/body. For the administration, a Myjector syringe with a 27G needle was used.
3) An NY-ESO-1 TCR-T cell suspension was administered to the tail vein. For the dosage, the number of viable NY-ESO-1 TCR-T cells was counted and a cell suspension adjusted to the cell density according to the group constitution table was administered once at 0.1 mL/body. For the administration, a Myjector syringe with a 27G needle was used.
4) For KHYG-1 cell administration solution and CD19 CAR-KHYG-1 cell administration solution, the number of viable

cells was counted and a cell suspension with the cell density being adjusted according to the group composition table was prepared and intratumorally administered twice a week at 0.1 mL/body (4 times in total). For the administration, a Myjector syringe with a 27G needle was used.

5) Birinapant was intraperitoneally administered at a dose of 10 mL/kg according to the group composition table. Tolinapant, LCL161, and AT406 were orally administered at a dose of 10 mL/kg according to the group composition table using a sonde in the same manner. For intraperitoneal administration, an insulin syringe with a 26G needle was used in place of the sonde. For oral administration, a needle was used in place of the sonde to administer each drug.

1.4.6 Measurement of Tumor

1.4.6.1 Measurement of Tumor Luminescence Value (photones/sec)

VivoGlo™-luciferin (produced by Promega) was dissolved in D-PBS(-) to achieve a concentration of 30 mg/mL and then cryopreserved at -20°C under light-shielded conditions. On the day of measurement, dissolved VivoGlo™-luciferin was diluted with an equivalent amount of D-PBS(-) and stored at room temperature under light-shielded conditions until use for testing. Based on the body weight measured in advance, VivoGlo™-luciferin was intraperitoneally administered in a volume of 10 mL/kg. 20 minutes after the administration, the luminescence value of the tumor was measured using the IVIS (trademark) imaging system. For the measurement, IVIS (trademark) Lumina X5 was used. In Figs. 1 to 5 and 7, total flux [p/s] on the ordinate indicates tumor luminescence values (photones/sec).

1.4.6.2 Measurement of Tumor Diameter

The tumor diameter was determined by measuring the short diameter and the long diameter using an electronic caliper (produced by Mitutoyo Corporation). After tumor cell transplantation, the tumor diameter was measured twice a week.

1.4.6.3 Measurement of Tumor Volume

Based on the measurement results of the tumor diameter, the tumor volume (TV: tumor volume) was calculated using the following formula:

$$TV = L \times W^2 \times 1/2$$

wherein

TV: tumor volume ($mm^3$)
L: long diameter (mm)
W: short diameter (mm)

1.4.7 Body Weight

The body weight was measured on the grouping day and the autopsy day, and twice a week during the administration period.

1.5 Evaluation Criteria

Tumor luminescence value or tumor volume
Body weight
Body weight change

1.6 Statistical Analysis Method

For each data item, summary statistics (mean and standard deviation and standard error) were calculated using Microsoft Excel 2013. Data with statistical analysis was performed using SAS software R9.4 (SAS Institute Japan). The

significance was defined as $p<0.05$.

## 2. Results

### 2.1 Test for Combination of IAP Antagonist and CD19 CAR-T

#### 2.1.1 Test for Combination of Birinapant and CD19 CAR-T

As compared to CD19 CAR-T alone, a combination of birinapant and CD19 CAR-T did not increase antitumor effects (Fig. 1).

#### 2.1.2 Test for Combination of Tolinapant and CD19 CAR-T

When tolinapant and CD19 CAR-T were used in combination, a significant combination effect was observed on day 7 and thereafter. When $1\times10^5$ cells of CD19 CAR-T was used in combination with tolinapant, an effect equivalent to the effect achieved by using $1\times10^6$ cells of CD19 CAR-T alone was observed on day 14, and better tumor regression was observed on day 14 and thereafter (Fig. 2).

#### 2.1.3 Test for Combination of Tolinapant and Cd19 Car-T Against a Low Volume of Tumor

When tolinapant and CD19 CAR-T were used in combination, a strong combination effect was observed. When $1 \times 10^5$ cells of CD19 CAR-T was used in combination with tolinapant, an effect equivalent to the effect achieved by using $1 \times 10^6$ cells of CD19 CAR-T alone was observed on day 17. When $2 \times 10^4$ cells of CD19 CAR-T was used in combination with tolinapant, an effect equivalent to the effect achieved by using $1\times10^6$ cells of CD19 CAR-T alone was observed on day 21. The results suggested that there is a possibility that this combination achieves at least 50-fold reduction in the number of CAR-T cells (Fig. 3).

#### 2.1.4 Test for Combination of CD19 CAR-T, LCL161, and AT406

When LCL161 and CD19 CAR-T were used in combination, a significant combination effect was observed on day 7 and thereafter. However, since LCL161 is highly toxic, at least half of the animals died due to LCL161. When $1 \times 10^5$ cells of CD19 CAR-T was used in combination with LCL161, an effect equivalent to the effect achieved by using $1 \times 10^6$ cells of CD19 CAR-T alone was observed on day 14 and thereafter. In terms of the highest efficacy as well, the efficacy achieved by the combined use of LCL161 was lower than the efficacy achieved by the combined use of tolinapant (Fig. 4).

On the other hand, when AT406 and CD19 CAR-T were used in combination, a combination effect was observed on day 10 and thereafter. When $1 \times 10^5$ cells of CD19 CAR-T was used in combination with AT406, an effect equivalent to the effect achieved by $1 \times 10^6$ cells of CD19 CAR-T alone was observed on day 14 and thereafter (Fig. 5).

### 2.2 Test for Tolinapant and Activated Integrin $\beta$7 CAR-T (aITGB7 CAR-T)

Tolinapant and $3 \times 10^4$ cells of aITGB7 CAR-T were used in combination, and their combination effect was evaluated for 21 days. A significant combination effect was observed. In a group to which $3 \times 10^4$ cells of aITGB7 CAR-T alone was administered, antitumor effects started to be exhibited on day 14 and thereafter. In contrast, when $3 \times 10^4$ cells of aITGB7 CAR-T was combined with tolinapant, tumor regression was clearly observed on day 11 and thereafter, and a significant synergistic effect was observed (Fig. 7).

### 2.3 Test for Combination of Tolinapant and NY-ESO-1 TCR-T

In terms of tumor volume on day 15, a significant difference was observed between a group to which a combination of $3 \times 10^6$ cells/body of NY-ESO-1 TCR-T and tolinapant was administered and groups to which only either NY-ESO-1 TCR-T or tolinapant was administered. A higher antitumor effect was observed in a group to which $1 \times 10^7$ cells/body of NY-ESO-1 TCR-T was administered alone than in a group to which $3 \times 10^6$ cells/body of NY-ESO-1 TCR-T was administered alone; and on day 7 and thereafter, the tumor volume was reduced to a level lower than that on day 0. However, in the group to which NY-ESO-1 TCR-T and tolinapant were both administered, a higher degree of tumor regression was confirmed and synergistic effects based on the combined use were shown.

2.4 Test for Combination of Tolinapant and CD19 CAR-KHYG-1

Tolinapant and CD19 CAR-KHYG-1 were used in combination, and their combination effect was evaluated for 17 days. A significant combination effect was observed. When either CD19 CAR-KHYG-1 or tolinapant was administered alone, no antitumor effects were observed. In contrast, when these two drugs were used in combination, a proliferation suppression effect was clearly shown on day 7 and thereafter, and significant combination effects were observed on day 17 (Fig. 9).

3 Conclusion

When an IAP antagonist, such as tolinapant, was administered alone, no effect on NALM6 (B-ALL) and MM.1S (MM) was observed. In contrast, significant, highly reproducible results were achieved by combination activity of tolinapant + CD19 CAR-T, tolinapant + activated integrin β7 CAR-T (aITGB7 CAR-T), tolinapant + NY-ESO-1 TCR-T, and tolinapant + CD19 CAR-KHYG-1. The other IAP antagonists, such as AT406 and LCL161, also showed a combination effect with CD19 CAR-T; however, LCL161 showed such toxicity as to confirm weight loss and death. In terms of the maximum effect as well, the combination with tolinapant was superior. Subsequently, although AT406 showed a combination effect profile similar to that of tolinapant, the speed at which the effect was exhibited is different. Accordingly, in terms of AUC achieved by combined use and the maximum tumor volume (luminescence value), tolinapant and AT406 were attempted to be differentiated from each other. The analysis shows that in terms of AUC in combination data up to day 17, a significance due to combined use of tolinapant could be observed. Similar results were also obtained on day 14 (Tables 9 and 10). In terms of the maximum tumor volume as well, a significant difference between tolinapant and AT406a could be obtained (Table 11).

The above results confirmed that the combination with tolinapant provides a higher drug efficacy-enhancement effect than combinations with any other IAP antagonist. This suggests that tolinapant is also significantly superior in terms of drug efficacy-enhancement effect achieved by the combined use with a modified immune cell, such as CAR-T cells, TCR-T cells, or CAR-NK cells.

It has also been confirmed that when CAR-T used in this Example was reacted with a target CAR antigen-expressing tumor cell (e.g., CD19-positive NALM6) in vitro, cytokine (e.g., TNF-α) was produced. Further, it has been confirmed that when the cytokine produced from CAR-T is present with tolinapant, cell death (e.g., apoptosis) is induced to antigen-negative tumor cells (e.g., CD19-negative NALM6) as well as antigen-expressing cells. This non-contact apoptosis induction to tumor cells is expected to exert an antitumor effect on tumor cells in which antigen escape has occurred or tumor cells metastasized to a site to which modified immune cells cannot make access.

Table 9: t-Test using AUC as an indicator (day 17)

| | AT406 | LCL161 | Birinapant |
|---|---|---|---|
| Tolinapant | <0.05 | <0.05 | <0.05 |

Table 10: t-Test using AUC as an indicator (day 14)

| | AT406 | LCL161 | Birinapant |
|---|---|---|---|
| Tolinapant | <0.05 | <0.05 | <0.05 |

Table 11: t-Test using the maximum tumor volume as an indicator

| | AT406 | LCL161 | Birinapant |
|---|---|---|---|
| Tolinapant | <0.05 | <0.05 | <0.05 |

**Claims**

**1.** A pharmaceutical composition comprising

a compound represented by formula (1):

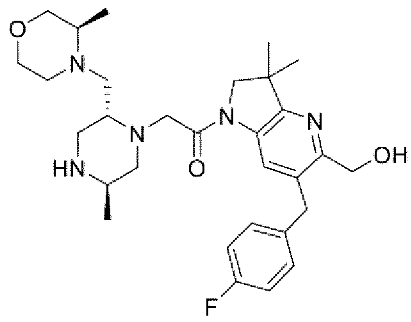

(1)

a pharmaceutically acceptable salt thereof, or
a pharmaceutically acceptable solvate thereof,
the pharmaceutical composition being administered in combination with an immune cell modified to express a chimeric antigen receptor or a recombinant T-cell receptor.

**2.** A pharmaceutical composition comprising an immune cell modified to express a chimeric antigen receptor or a recombinant T-cell receptor,

the pharmaceutical composition being administered in combination with
a compound represented by the following formula (1):

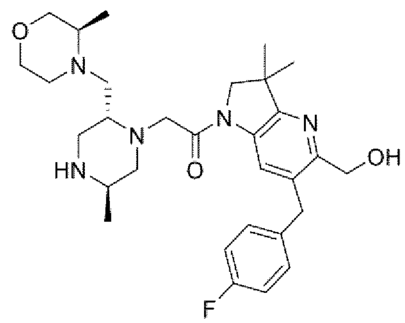

(1)

,

a pharmacologically acceptable salt thereof, or
a pharmacologically acceptable solvate thereof.

**3.** The pharmaceutical composition according to claim 1 or 2, wherein the chimeric antigen receptor or the recombinant T-cell receptor specifically recognizes an antigen selected from the group consisting of CD19, integrin β7, and NY-ESO-1.

**4.** The pharmaceutical composition according to claim 1 or 2, wherein the chimeric antigen receptor or the recombinant T-cell receptor specifically recognizes activated integrin β7.

**5.** The pharmaceutical composition according to any one of claims 1 to 4, wherein

the immune cell is a T cell or NK cell modified to express a chimeric antigen receptor, and
the chimeric antigen receptor comprises

(1) an extracellular domain capable of recognizing an antigen,
(2) a transmembrane domain,
(3) an intracellular signaling domain of a costimulatory molecule, and
(4) an intracellular signaling domain of CD3ζ.

**6.** The pharmaceutical composition according to claim 5, wherein the extracellular domain is capable of recognizing CD19 or integrin β7.

7. The pharmaceutical composition according to claim 5 or 6, wherein the transmembrane domain is a transmembrane domain of CD28 or CD8.

8. The pharmaceutical composition according to any one of claims 5 to 7, wherein the costimulatory molecule is CD2, CD4, CD5, CD8α, CD8β, CD28, CD134 (OX40), CD137 (4-1BB), or CD278 (ICOS).

9. The pharmaceutical composition according to claim 1 or 2, wherein

the immune cell is a T cell or NK cell modified to express a recombinant T-cell receptor; and
the recombinant T-cell receptor has a variable domain capable of recognizing NY-ESO-1.

10. The pharmaceutical composition according to claim 1 or 2, wherein the immune cell is

a T cell modified to express a chimeric antigen receptor that specifically recognizes CD19,
a T cell modified to express a chimeric antigen receptor that specifically recognizes integrin β7,
an NK cell modified to express a chimeric antigen receptor that specifically recognizes CD19, or
a T cell modified to express a recombinant T-cell receptor that specifically recognizes NY-ESO-1.

11. The pharmaceutical composition according to any one of claims 1 to 10 for use in the prevention and/or treatment of cancer.

12. The pharmaceutical composition according to any one of claims 1 to 11, wherein the compound represented by formula (1), a pharmaceutically acceptable salt thereof, or a pharmaceutically acceptable solvate thereof is administered at a dose of 10 to 180 mg/day.

13. The pharmaceutical composition according to any one of claims 1 to 12, wherein the immune cell is administered at a dose of $1 \times 10^3$ to $1 \times 10^8$ cells (viable cell count)/kg body weight/day.

14. A kit comprising a first agent and a second agent,

the first agent comprising

a compound represented by formula (1):

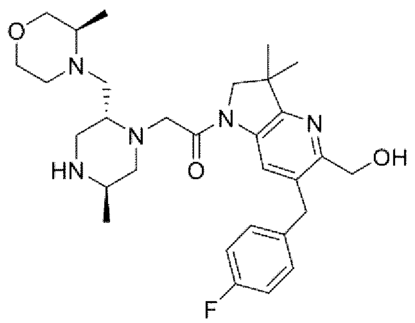

(1)

a pharmacologically acceptable salt thereof, or
a pharmaceutically acceptable solvate thereof, and

the second agent comprising an immune cell modified to express a chimeric antigen receptor or a recombinant T-cell receptor.

15. A method for preventing and/or treating a cancer, comprising administering to a subject in need of prevention and/or treatment of the cancer

a compound represented by formula (1):

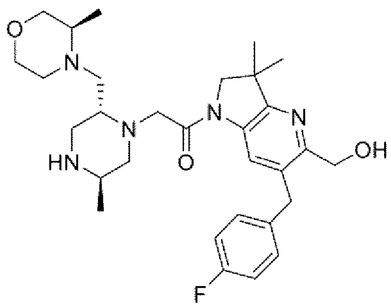

(1)

,

a pharmacologically acceptable salt thereof, or
a pharmaceutically acceptable solvate thereof, in combination with
an immune cell modified to express a chimeric antigen receptor or a recombinant T-cell receptor.

**16.** Use of a compound represented by formula (1)

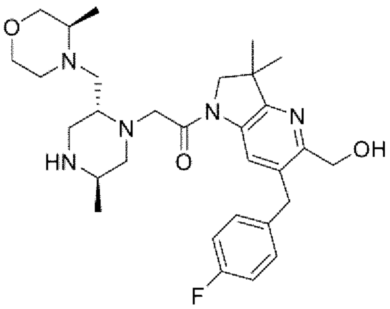

(1)

,

a pharmacologically acceptable salt thereof, or
a pharmaceutically acceptable solvate thereof
in preparing a medicament for administration in combination with an immune cell modified to express a chimeric antigen receptor or a recombinant T-cell receptor.

**17.** Use of an immune cell modified to express a chimeric antigen receptor or a recombinant T-cell receptor in preparing a medicament for administration in combination with a compound represented by formula (1):

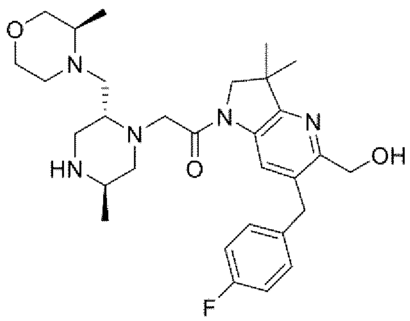

(1)

a pharmacologically acceptable salt thereof, or
a pharmaceutically acceptable solvate thereof.

Fig. 1

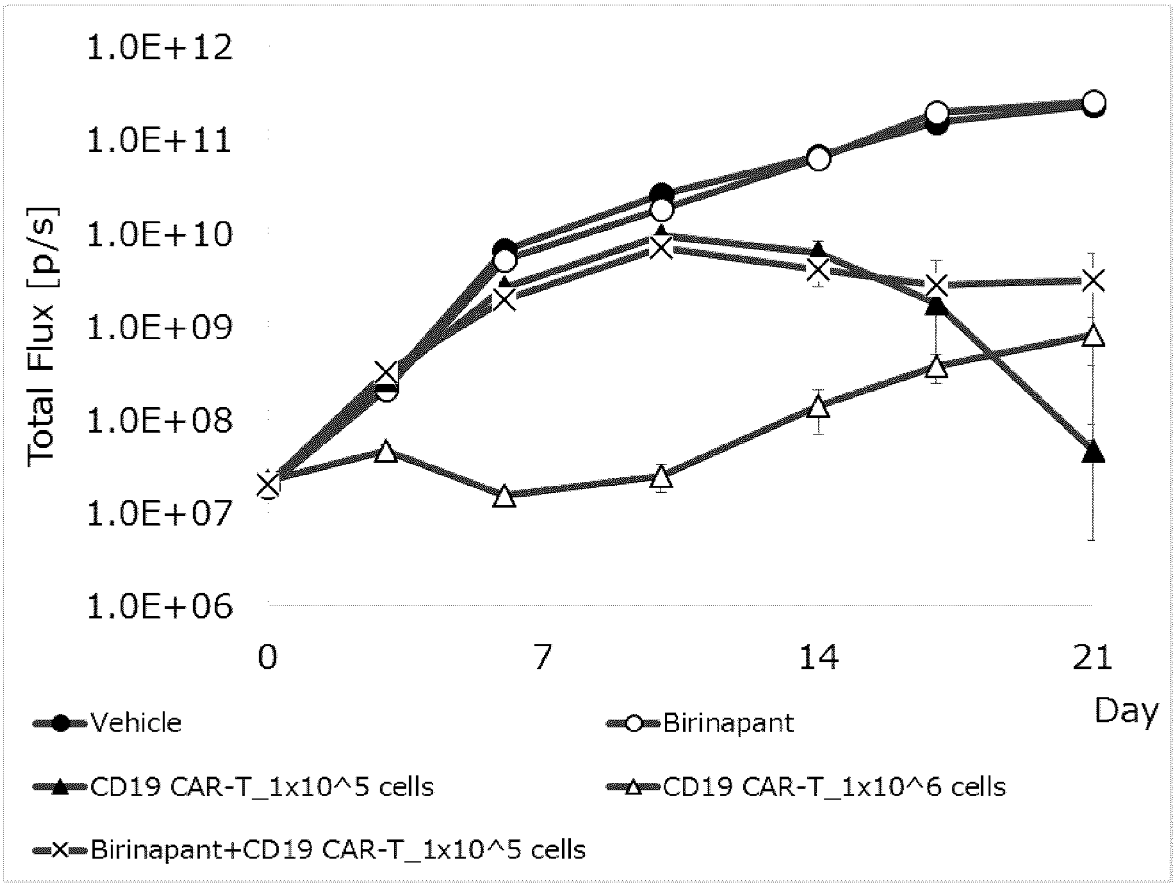
1.0E+12
1.0E+11
1.0E+10
1.0E+09
1.0E+08
1.0E+07
1.0E+06
Total Flux [p/s]
0
7
14
21
Day
Vehicle
Birinapant
CD19 CAR-T_1x10^5 cells
CD19 CAR-T_1x10^6 cells
Birinapant+CD19 CAR-T_1x10^5 cells

Fig. 2

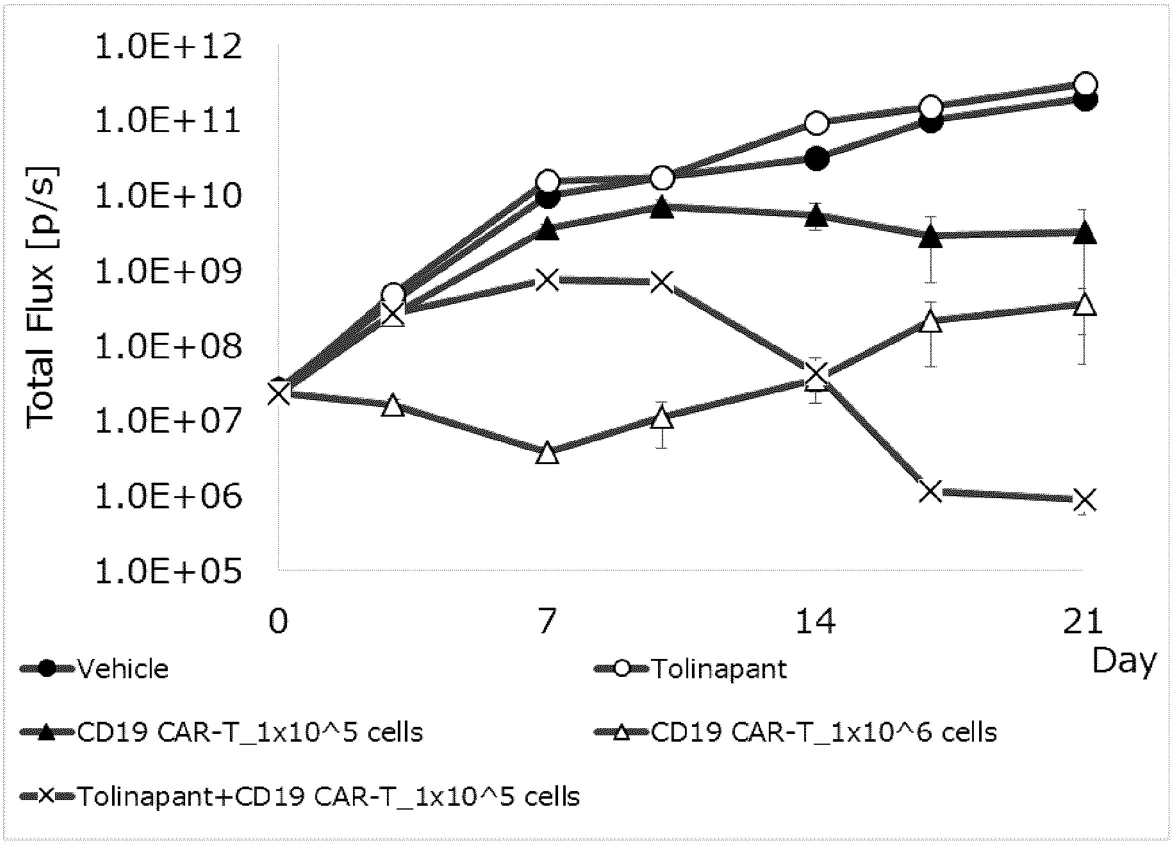
Total Flux [p/s]
1.0E+12
1.0E+11
1.0E+10
1.0E+09
1.0E+08
1.0E+07
1.0E+06
1.0E+05
0
7
14
21
Day
Vehicle
Tolinapant
CD19 CAR-T_1x10^5 cells
CD19 CAR-T_1x10^6 cells
Tolinapant+CD19 CAR-T_1x10^5 cells

Fig. 3

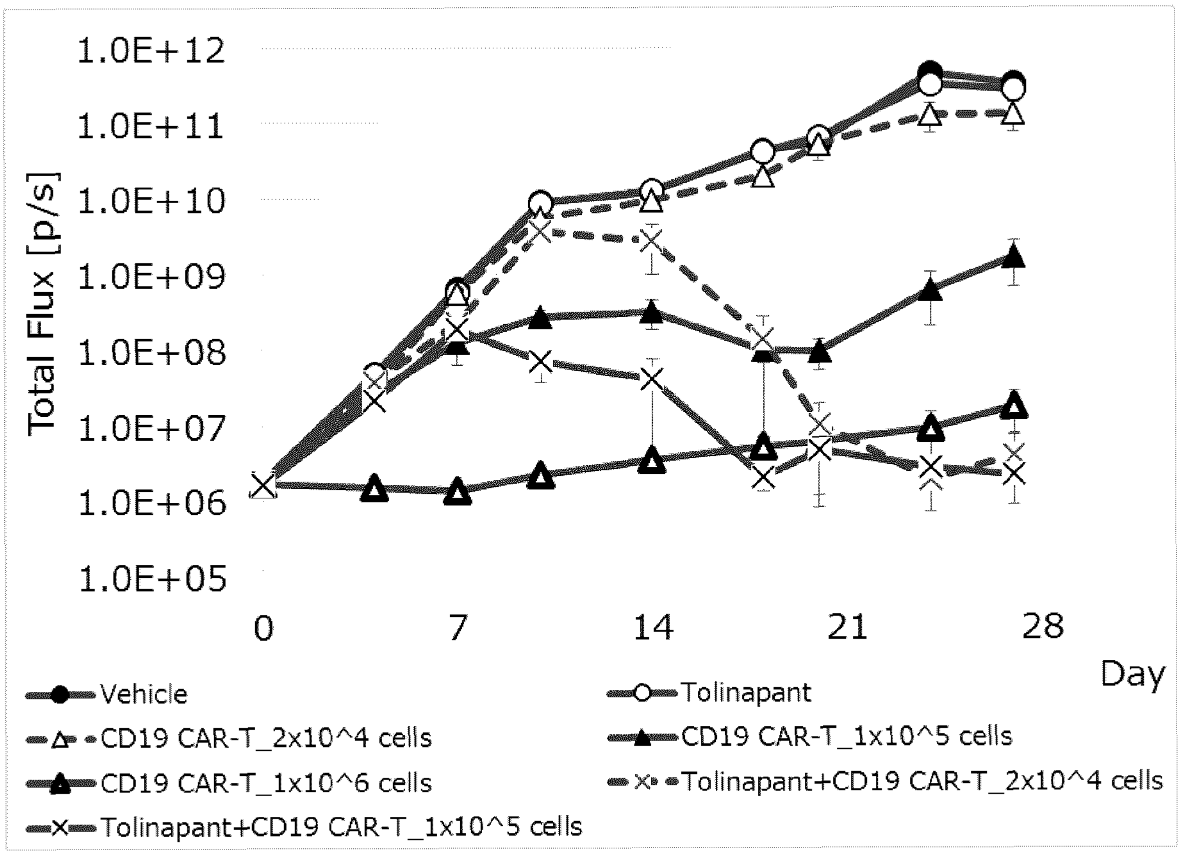
Total Flux [p/s]
1.0E+12
1.0E+11
1.0E+10
1.0E+09
1.0E+08
1.0E+07
1.0E+06
1.0E+05
0
7
14
21
28
Day
Vehicle
Tolinapant
CD19 CAR-T_2x10^4 cells
CD19 CAR-T_1x10^5 cells
CD19 CAR-T_1x10^6 cells
Tolinapant+CD19 CAR-T_2x10^4 cells
Tolinapant+CD19 CAR-T_1x10^5 cells

Fig. 4

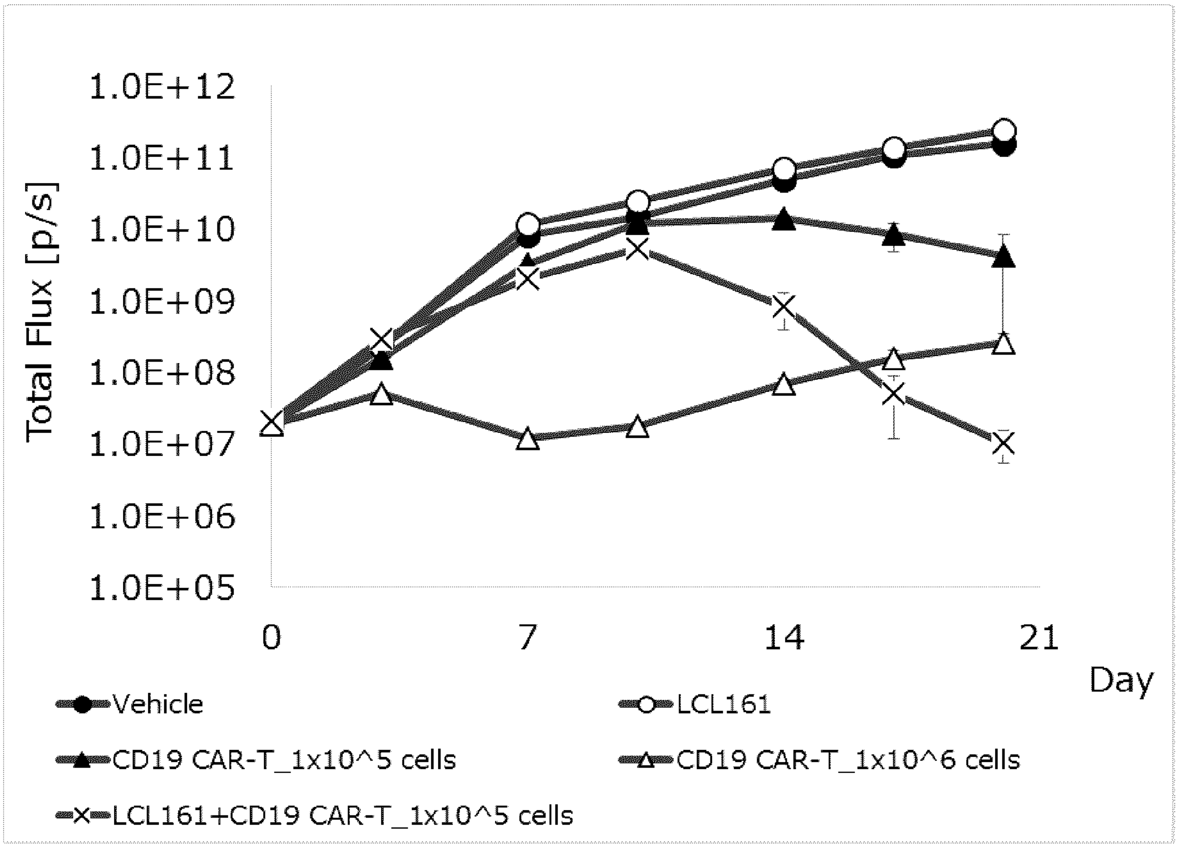
Total Flux [p/s]
1.0E+12
1.0E+11
1.0E+10
1.0E+09
1.0E+08
1.0E+07
1.0E+06
1.0E+05
0
7
14
21
Day
Vehicle
LCL161
CD19 CAR-T_1x10^5 cells
CD19 CAR-T_1x10^6 cells
LCL161+CD19 CAR-T_1x10^5 cells

Fig. 5

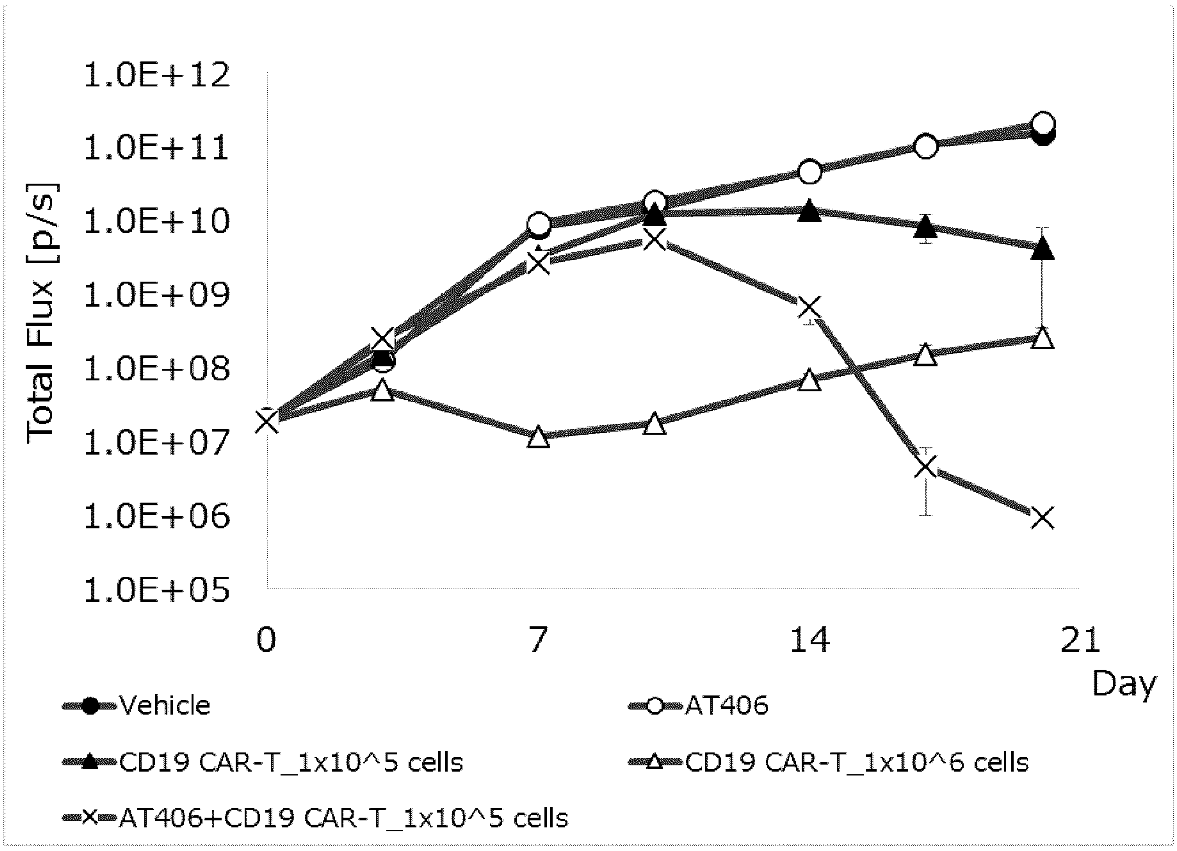
Total Flux [p/s]
1.0E+12
1.0E+11
1.0E+10
1.0E+09
1.0E+08
1.0E+07
1.0E+06
1.0E+05
0
7
14
21
Day
Vehicle
AT406
CD19 CAR-T_1x10^5 cells
CD19 CAR-T_1x10^6 cells
AT406+CD19 CAR-T_1x10^5 cells

Fig. 6

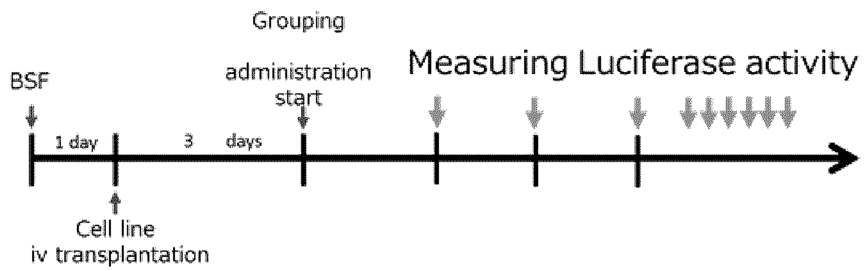
Grouping
administration start
Measuring Luciferase activity
BSF
1 day
3 days
Cell line
iv transplantation

Fig. 7

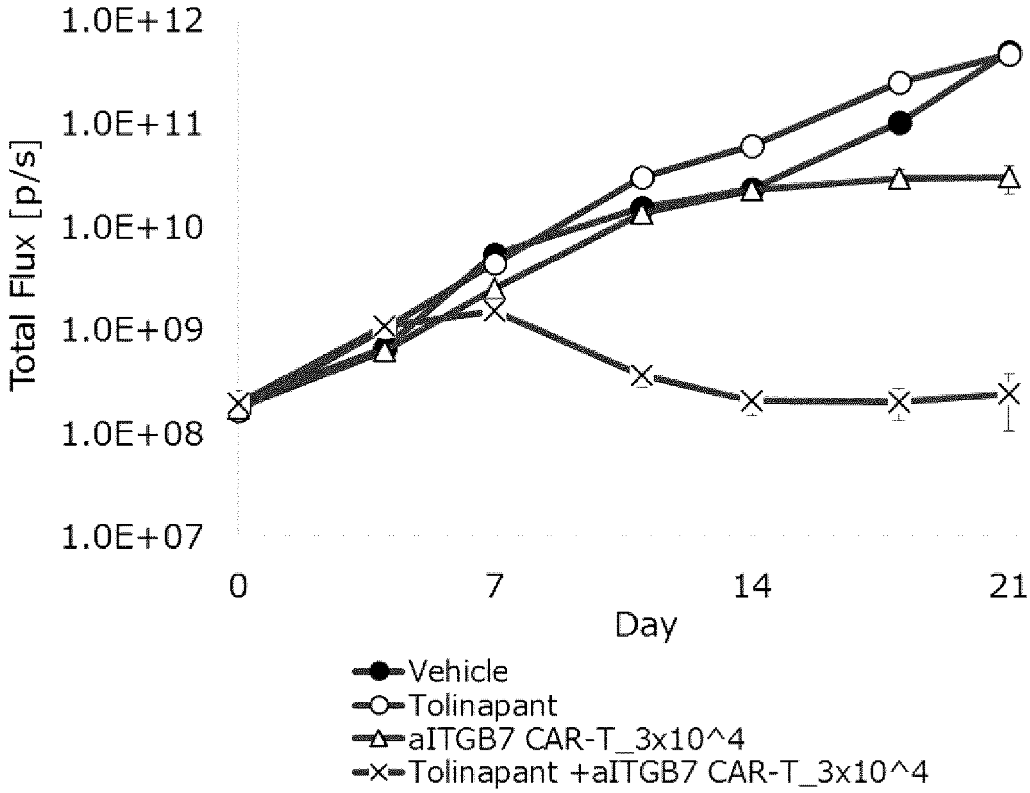
1.0E+12
1.0E+11
1.0E+10
1.0E+09
1.0E+08
1.0E+07
Total Flux [p/s]
0
7
14
21
Day
Vehicle
Tolinapant
aITGB7 CAR-T_3x10^4
Tolinapant +aITGB7 CAR-T_3x10^4

Fig. 8

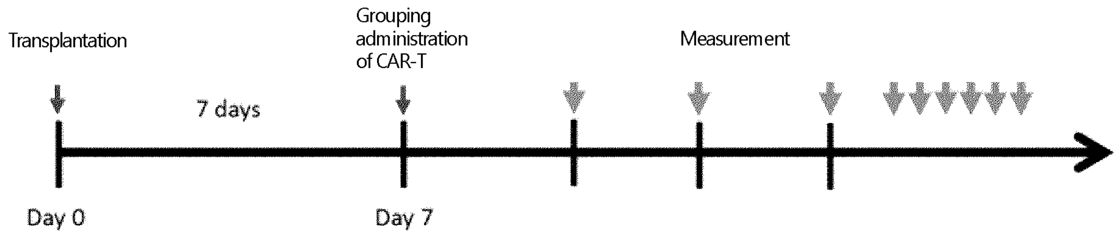


Fig. 9

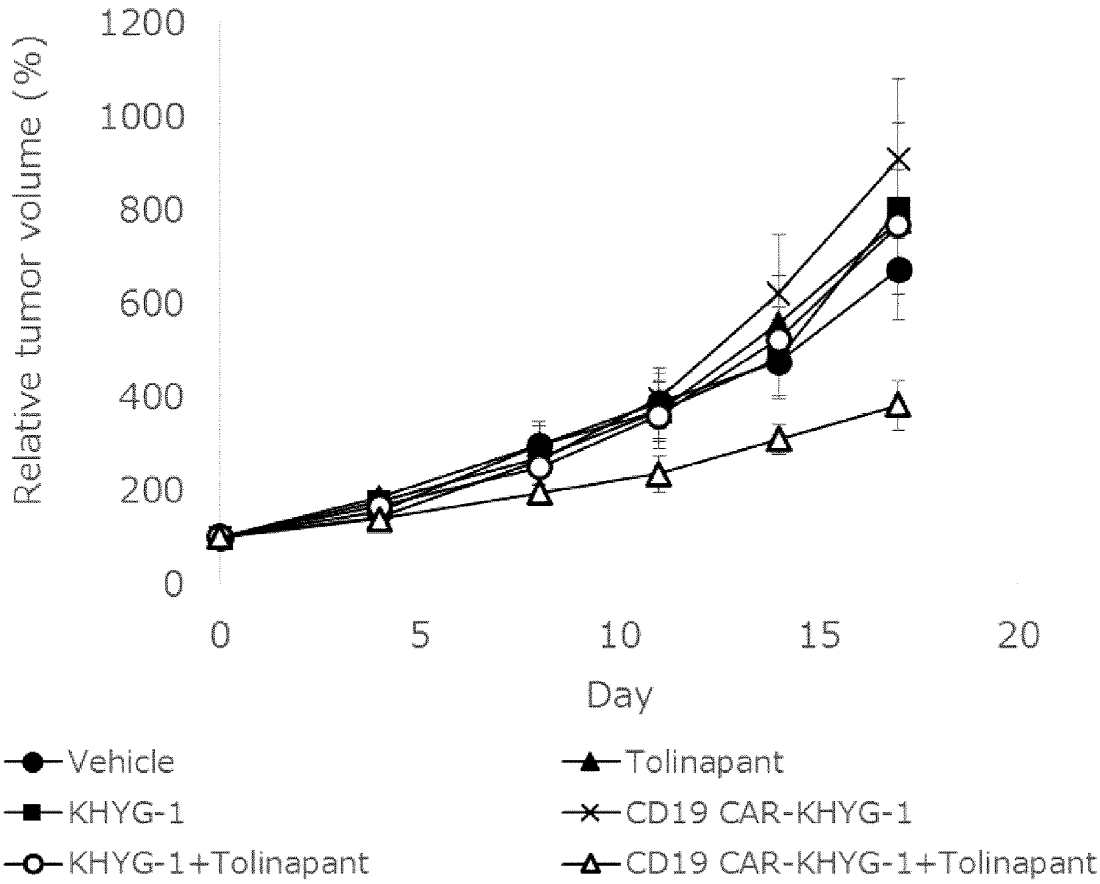


Fig. 10

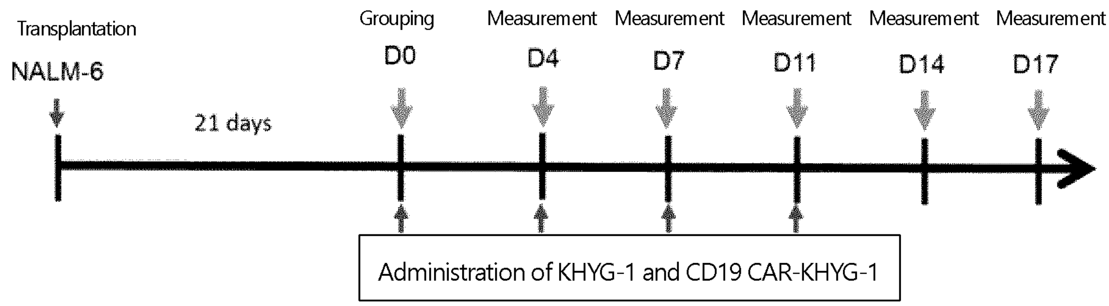

## INTERNATIONAL SEARCH REPORT

International application No.
**PCT/JP2022/031444**

**A. CLASSIFICATION OF SUBJECT MATTER**

***A61K 31/5377***(2006.01)i; ***A61K 35/17***(2015.01)i; ***A61P 35/00***(2006.01)i; ***A61P 43/00***(2006.01)i
FI: A61K31/5377 ZNA; A61K35/17 Z; A61P35/00; A61P43/00 121

According to International Patent Classification (IPC) or to both national classification and IPC

**B. FIELDS SEARCHED**

Minimum documentation searched (classification system followed by classification symbols)

A61K31/5377; A61K35/17; A61P35/00; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | XIAO, R. et al. P289 Dual cIAP1/XIAP inhibitor ASTX660 synergizes with radiation therapy and PD-1 blockade to enhance anti-tumor immunity. Journal for ImmunoTherapy of Cancer. 2017, vol. 5 (Suppl 2), pp. 117 of 244, 148 of 244<br>in particular, Methods, Results | 1-17 |
| Y | YE, W. et al. ASTX660. an antagonist of cIAP1/2 and XIAP, increases antigen processing machinery and can enhance radiation-induced immunogenic cell death in preclinical models of head and neck cancer. ONCOIMMUNOLOGY. 2020, vol. 9, no. 1, e1710398 (pp. 1-13), Supplementary Figures (S1-S8), Supplementary Methods<br>in particular, p. 5, right column, second paragraph, fig. 4 | 1-17 |
| Y | XIAO, R. et al. Antagonist of cIAP1/2 and XIAP enhances anti-tumor immunity when combined with radiation and PD-1 blockade in a syngeneic model of head and neck cancer. ONCOIMMUNOLOGY. 2018, vol. 7, no. 9, e1471440 (pp. 1-12)<br>in particular, p. 5, left column, fig. 6 | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 October 2022** | **01 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**

International application No. **PCT/JP2022/031444**

**C. DOCUMENTS CONSIDERED TO BE RELEVANT**

| Category* | Citation of document, with indication, where appropriate, of the relevant passages | Relevant to claim No. |
|---|---|---|
| Y | FERRARI, N. et al. 109 The non-peptidomimetic cIAP1/2 and XIAP antagonist ASTX660 promotes an anti-tumour immune response in T-cell lymphoma. European Journal of Cancer. 2020, vol. 138, Supplement 2, p. S28<br>in particular, Materials and Methods, Results | 1-17 |
| Y | 編集河本宏他. 実験医学 増刊. 2020, vol. 38, no. 17, pp. 25-34, 107-121, (pp.2817-2826, 2899-2913), (KAWAMOTO, Hiroshi, TSUJI, Masahiro eds. Experimental Medicine (Suppl.).)<br>in particular, p. 27, table 1, p. 109, fig. 1, p. 119, tables | 1-17 |
| Y | 保仙直毅. 9. CAR-T細胞療法. 実験医学 増刊. 2019, vol. 37, no. 15, pp. 164-169, (pp. 2556-2561), (HOSEN, Naoki. Experimental Medicine (Suppl.)), non-official translation (9. CAR-T Cell Therapy.)<br>in particular, p. 165, fig. 1, p. 168, fig. 4 | 1-17 |
| Y | MICHIE, J. et al. Antagonism of IAPs Enhances CAR T-cell Efficacy. Cancer Immunology Research. 2019, vol. 7, no. 2, pp. 183-192<br>in particular, p. 184 (Generation of CAR T cells) | 1-17 |
| Y | JP 2019-504026 A (IMMUNOCORE LTD.) 14 February 2019 (2019-02-14)<br>abstract, claims, paragraphs [0001]-[0008] | 1-17 |
| P, X | WO 2021/202801 A1 (FRED HUTCHINSON CANCER RESEARCH CENTER) 07 October 2021 (2021-10-07)<br>claims, paragraphs [0110]-[0162], [0247], fig. 9B | 1-3, 5, 7-17 |
| P, Y | claims, paragraphs [0110]-[0162], [0247], fig. 9B | 4, 6 |
| P, Y | FERRARI, N. et al. Antagonism of inhibitors of apoptosis proteins reveals a novel, immune response-based therapeutic approach for T-cell lymphoma. Blood Advances. October 2021, vol. 5, no. 20, pp. 4003-4016, Supplementary Materials and Methods (pp. 1-14), Supplementary Figures (S1-S6), Supplementary Figure Legends (pp. 1-3)<br>in particular, p. 4007, right column, third paragraph to p. 4010, right column, first paragraph, fig. 3, S3 | 1-17 |

Form PCT/ISA/210 (second sheet) (January 2015)

## INTERNATIONAL SEARCH REPORT

International application No.
**PCT/JP2022/031444**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "Annex C/ST.25 text file format" should be read as "ST.26 format".

**INTERNATIONAL SEARCH REPORT**
**Information on patent family members**

International application No.
**PCT/JP2022/031444**

| Patent document cited in search report | Publication date (day/month/year) | Patent family member(s) | Publication date (day/month/year) |
|---|---|---|---|
| JP 2019-504026 A | 14 February 2019 | US 2019/0002523 A1<br>abstract, claims, paragraphs [0001]-[0014]<br>WO 2017/109496 A1<br>EP 3394094 A1<br>KR 10-2018-0090374 A<br>CN 109476723 A | |
| WO 2021/202801 A1 | 07 October 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015092420 A
- WO 1993019163 A
- US 4694778 A
- WO 2017026331 A
- WO 2008153029 A
- US 20180230212
- US 20180230216


### Non-patent literature cited in the description


- *Science,* 1988, vol. 242, 423-442
- *Nature,* 1989, vol. 334, 54454
- *Science,* 1988, vol. 242, 1038-1041
- *Molecular Immunology,* 1997, vol. 34 (16-17), 1157-1165
- *Nature Medicine,* 2007, vol. 23 (12), 1436
- *Nature,* 1989, vol. 338, 383-384
- *J Immunol.,* 1999, vol. 162 (2), 897-902
- *Mol Cancer Ther.,* July 2018, vol. 17 (7), 1381-1391
- *Oncoimmunology,* 09 January 2020, vol. 9 (1), 1710398
- **JESSICA MICHIE et al.** *Cancer Immunol Res.,* 2019, vol. 7 (2), 183-192
- **PETER J HOUGHTON et al.** *Pediatr Blood Cancer.,* 2012, vol. 58 (4), 636-639
- **MELISSA K BRUNCKHORST et al.** *Cancer Biology & Therapy.,* 2012, vol. 13 (9), 804-811
- **QIAN CAI et al.** *Med Chem.,* 2011, vol. 54 (8), 2714-2726
- **NAKAZAWA T et al.** *Anticancer Res.,* 2020, vol. 40 (6), 3231-3237
- **MINSUNG KIM et al.** *PLoS One.,* 2018, vol. 13 (6), e0198347